# EUROPÄISCHE PATENTSCHRIFT

(11) **EP 3 410 965 B1**
(45) Veröffentlichungstag und Bekanntmachung des Hinweises auf die Patenterteilung: **04.10.2023**
(21) Anmeldenummer: 17702632.5
(22) Anmeldetag: 02.02.2017
(51) Int. Cl.: A61B 17/70

(54) **INSTRUMENT ZUM FÜHREN EINES STABS IN EINE IMPLANTATAUFNAHME**
INSTRUMENT FOR GUIDING A ROD INTO AN IMPLANT HOLDER
INSTRUMENT POUR GUIDER UNE TIGE DANS UN LOGEMENT D'IMPLANT

(30) Priorität: 02.02.2016 DE 102016101822; 11.04.2016 DE 102016106608
(43) Veröffentlichungstag der Anmeldung: 12.12.2018
(73) Patentinhaber: Aesculap AG, 78532 Tuttlingen (DE)
(72) Erfinder: FISCHER, Kay, 78532 Tuttlingen (DE); KRÜGER, Sven, 78647 Trossingen (DE)
(74) Vertreter: Winter, Brandl - Partnerschaft mbB
(86) Internationale Anmeldenummer: PCT/EP2017/052234
(87) Internationale Veröffentlichungsnummer: WO 2017/134154

(56) Entgegenhaltungen:
- EP-A2- 2 878 276
- WO-A2-2013/009493
- US-A1- 2012 191 144
- US-A1- 2014 276 894
- US-A1- 2014 276 895

## Beschreibung

Die vorliegende Erfindung betrifft ein Instrument zum Relativpositionieren und/oder Führen und/oder Einführen eines Stabs in eine Aufnahme eines Implantats, insbesondere einer Pedikelschraube, umfassend eine Kopplungseinheit zum Koppeln des Instruments mit dem Implantat, insbesondere mit einem Kopf der Pedikelschraube, eine mit einem Außengewinde versehene und zur Kopplungseinheit in axialer Richtung positionierbare Gewindestange und zumindest ein zur Kopplungseinheit axialfest angeordnetes Innengewindesegment, das gegenüber der Gewindestange in radialer Richtung positionierbar ist und durch ein eine Radialkraft aufbringendes Vorspannelement in Richtung der Gewindestange vorgespannt ist, wobei das Innengewindesegment durch Radialpositionierung mit dem Außengewinde der Gewindestange in Eingriff bzw. außer Eingriff bringbar ist.

Derartige Instrumente werden zum Beispiel bei offenen Wirbelsäulen-Operationen eingesetzt, um mittels eines Stabs eine feste oder rigide Verbindung zwischen in verschiedene Wirbel eingeschraubte Pedikelschrauben zu erstellen. Das Instrument dient dazu, Stäbe, über die benachbarte Pedikelschrauben zueinander fixiert werden, in deren jeweilige Tulpe einzuführen und in dieser Position zu halten, damit der Stab dort mit einer Set-Screw gesichert werden kann.

In Fällen, bei denen die Distanz des Stabs zur Tulpe gering ist, wird in der Regel mit einem Rod Pusher, einem einfachen Hebelinstrument, gearbeitet, um den Stab in die Tulpe zu drücken. In Fällen, bei denen der Stab weit über der Tulpe platziert und von dieser beabstandet ist, z.B. bedingt durch eine Spondylolisthese (Wirbelgleiten), sind unter Umständen große Kräfte erforderlich, um den Stab in die Tulpe zu zwingen bzw. einen abgeglittenen Wirbelkörper in die gewünschte Position zu ziehen. Hierfür werden in der Regel Rod Persuader verwendet.

Bekannt ist ein Instrument mit Pistolengriff und Ratschenmechanismus, bei dem die Ratsche zum Fixieren eines beim Einführen des Stabs zurückgelegten Weges dient. Mit diesem Instrument können Kräfte von mittlerer Größe übertragen werden. Es ist aber von Nachteil, dass bedingt durch den Ratschenmechanismus keine stufenlose Verstellung möglich ist.

Bei einem weiteren bekannten Instrument erfolgt ein Positionieren des Stabs in Richtung eines Pedikelschraubenkopfs ausschließlich durch eine Schraubbetätigung des Instruments, das so in der Lage ist, durch sein Gewinde enorm hohe Kräfte aufzubringen. Des Weiteren ist eine stufenlose Verstellung möglich. Im Falle, dass das Gewinde eines Rod Persuaders selbsthemmend ausgebildet ist, kann jede beliebige Position gehalten werden. Nachteilig hierbei ist eine aufwendige und mühevolle Verstellung über weiter Zustellstrecken.

Schließlich ist ein Rod Persuader mit Zangendesign bekannt. Dieses Instrument kann aufgrund seiner mehrfachen Übersetzung beim Einführen des Stabs einen großen Weg zurücklegen, ist jedoch nicht in der Lage, derart hohe Kräfte wie ein vorstehend beschriebener Rod Persuader zu übertragen.

Das Funktionsprinzip der drei vorstehend genannten Typen ist ähnlich. Ein Teil des Instruments greift den Kopf einer Pedikelschraube und ein anderer Teil des Instruments stützt sich auf einen in den Kopf einzudrückenden Stab ab. Die beiden Teile werden über einen der vorstehend beschriebenen Mechanismen einander angenähert, so dass der Stab in die Tulpe des Pedikelschraubenkopfs gedrückt wird bzw. die Schraube zum Stab hin gezogen wird.

Ein bekanntes Instrument mit Schraubbetätigung besitzt eine Implantataufnahme, eine Gewindehülse und einen vorderen Handgriff. Implantataufnahme, Gewindehülse und Handgriff sind dreh- und axialfest zueinander angeordnet oder ausgebildet. Das Instrument weist des Weiteren einen Stabdrücker und eine Gewindestange auf, die ebenfalls axialfest aber zueinander drehbar miteinander gekoppelt sind. Beide Bauteile sind im Instrument relativ zur Implantataufnahme verschiebbar gelagert. Die Gewindestange ist an ihrem distalen, vom Stabdrücker abgewandten Ende mit einem Handgriff versehen und steht mit der Gewindehülse in Eingriff. Durch eine Drehbewegung des Handgriffs kann sie daher in die Gewindehülse hinein- und aus dieser herausgeschraubt werden. Eine dabei ausgeführte Translation wird infolge der vorstehend beschriebenen Kopplung mit dem Stabdrücker auf diesen übertragen, wobei der Stabdrücker jedoch nicht rotiert. Im Resultat erfolgt durch Ein- und Ausschrauben der Gewindestange in bzw. aus der Gewindehülse eine Axialpositionierung von Implantataufnahme und Stabdrücker relativ zueinander.

Die mögliche relative Axialpositionierung von Implantataufnahme und Stabdrücker bestimmt die Distanz, über die mittels des Instruments ein Stab in eine Pedikelschraubentulpe gedrückt werden kann. Üblicherweise ist diese Distanz größer als 25mm, z.B. 45mm. Je nach Gewindesteigung ist eine entsprechende Anzahl an Umdrehungen erforderlich, um den Stab in die Tulpe zu drücken. Es ist von Nachteil, dass das dazu erforderliche Drehen, Umgreifen und Weiterdrehen ermüdend und zeitraubend ist und den Ablauf einer Operation verzögert, zumal dieses Vorgehen u.U. bei mehreren Pedikelschrauben wiederholt werden muss.

Eine bekannte Lösung für dieses Problem stellt ein zum Beispiel in der US 2015/0100097 A1 oder ein in der US 2015/0100098 A1 offenbarter Rastmechanismus dar. Dort greift eine Gewindestange in eine in radialer Richtung relativpositionierbare und gefederte Gewindeschale bzw. einen Gewindeabschnitt ein. Bei axialem Druck auf die Gewindestange verschiebt sich die Gewindeschale / der Gewindeabschnitt gegen die Federvorspannung in radiale Richtung (federt ein), so dass die Gewindestange ohne Schraubbewegung in axialer Richtung vorgeschoben werden kann, bis ein durch den Stab ausgeübter Gegendruck zu groß wird. Ab diesem Punkt kann eine weitere Axialpositionierung durch Schrauben erfolgen, wobei hohe Kräfte auf den Stab übertragen werden können. Sobald der Stab die gewünschte Endposition erreicht hat, wird eine Set-Screw in die Tulpe geschraubt und der Stab derart fixiert. Durch Druck auf einen Entriegelungsknopf, der mit der Gewindeschale bzw. dem Gewindeabschnitt wirkverbunden ist, werden die Gewinde voneinander gelöst und außer Eingriff gebracht. Die Gewindestange wird so freigeben und kann zurückgezogen werden. Vorteile dieses Mechanismus sind ein weitgehend ermüdungsfreies Arbeiten und Zeitersparnis, da nicht die gesamte Axialpositionierung durch Schrauben erfolgen muss. Da diese Instrumente aber in der Lage sind, Druckkräfte von mehreren 1000N auszuüben, stellt ein Entkoppeln von Gewindestange und Gewindeschale unter Druck in nachteiliger Weise eine Gefahr dar, umliegende Strukturen oder OP-Personal zu verletzten. US 2012/191144 A1 offenbart weitere Beispiele von Instrumenten zum Führen eines Stabs in eine Aufnahme einer Pedikelschraube. Die Instumenten umfassen eine Kopplungseinheit zum Koppeln des Instruments mit dem Implantat, insbesondere mit einem Kopf der Pedikelschraube, sowie eine mit einem Außengewinde versehene und zur Kopplungseinheit in axialer Richtung positionierbare Gewindestange. Darüber hinaus verfügt die Kupplungseinheit über eine Entriegelungsvorrichtung, mit der die axiale Position der Gewindestange ver- und entriegelt werden kann.

Vor diesem Hintergrund liegt der vorliegenden Erfindung die Aufgabe zugrunde, ein Instrument zum Relativpositionieren, Führen und Einführen eines Stabs in eine Aufnahme eines Implantats, insbesondere einer Pedikelschraube bereitzustellen, mit dem hohe Druckkräfte zwischen Stab und Implantat übertragen werden können, und das auch für große Anfangsabstände von Stab und Implantat geeignet ist. Das Instrument soll eine einfach zu betätigende und sichere Schnellverstellung sowohl beim Zustellen als auch beim Lösen ermöglichen, so dass eine Gefährdung und Verletzung von an das Instrument angrenzenden Strukturen sowie von OP-Personal ausgeschlossen oder zumindest minimiert ist. Des Weiteren soll das Instrument zu Reinigungszwecken einfach zu demontieren sein, eine unbeabsichtigte Demontage soll aber stets sicher vermieden werden.

Diese Aufgabe wird nach der vorliegenden Erfindung gelöst durch ein Instrument zum Relativpositionieren und/oder Führen und/oder Einführen eines Stabs, zum Beispiel einer Wirbelstange, in eine Aufnahme eines Implantats, insbesondere einer Pedikelschraube, zum Beispiel in einen Kopf einer Pedikelschraube, umfassend eine Kopplungseinheit zum (axialfesten) Koppeln des Instruments mit dem Implantat bzw. mit einer Implantataufnahme bzw. mit der Pedikelschraube bzw. mit deren Kopf, eine mit einem Außengewinde versehene und zur Kopplungseinheit in axialer Richtung positionierbare Gewindestange und zumindest ein zur Kopplungseinheit axialfest angeordnetes Innengewindesegment, das gegenüber der Gewindestange in radialer Richtung positionierbar ist und durch ein eine Radialkraft aufbringendes Vorspannelement in Richtung der Gewindestange vorgespannt ist, wobei das Innengewindesegment durch Radialpositionierung mit dem Außengewinde der Gewindestange in Eingriff bzw. außer Eingriff bringbar ist, welches Instrument dadurch gekennzeichnet ist, dass Gewindeflanken der Gewindestange und Gewindeflanken des Innengewindesegments, die einander zugewandt sind, jeweils einen Hinterschnitt aufweisend ausgebildet sind, und wobei wenigstens ein Entriegelungselement in tangentialer Richtung des Innengewindesegments angeordnet und in radialer Richtung durch nutzerseitige Betätigung positionierbar ist.

Bei der Beschreibung der Erfindung verwendete Richtungsangaben, wie axial, radial und tangential, sind auf die Gewindestange und deren Orientierung bezogen. Die Bezeichnungen "proximal" und "distal" sind auf den Arbeitsbereich des Instruments, also den Rumpf eines Patenten, bezogen. In diesem Sinne bedeutet distal "vom Rumpf des Patienten entfernt", während proximal "zum Rumpf des Patienten hin gelegen" oder "zum Rumpf des Patienten hin verlaufen" bedeutet. Bei bestimmungsgemäßer Nutzung des Instruments ist dessen Längsachse, d.h. auch die Längsachse der Gewindestange, im Wesentlichen in Richtung der Längsachse einer Pedikelschraube ausgerichtet. Man kann daher sagen, dass das Instrument wie auch die Gewindestange jeweils ein proximales und ein distales Ende aufweisen.

Bei in radialer Richtung zur Gewindestange positioniertem Innengewindesegment, im Folgenden auch erste Position bezeichnet, stehen das Außengewinde der Gewindestange und das Innengewinde des Innengewindesegments miteinander in Eingriff. Bei in radialer Richtung von der Gewindestange fort positioniertem Innengewindesegment, im Folgenden auch als zweite Position bezeichnet, sind sie außer Eingriff gebracht. In der zweiten Position ist daher die Gewindestange relativ zum Innengewindesegment frei axialpositionierbar, das heißt axial sowohl in proximaler als auch in distaler Richtung positionierbar, insbesondere durch nutzerseitige Einwirkung verschiebbar. In der ersten Position ist die Gewindestange relativ zum Innengewindesegment zumindest in eine axiale Richtung nicht frei axialpositionierbar, da erfindungsgemäß Gewindeflanken der Gewindestange und Gewindeflanken des Innengewindesegments, die einander zugewandt sind, jeweils einen Hinterschnitt aufweisend ausgebildet sind. Die jeweils gegenüberliegenden Gewindeflanken von Gewindestange und Innengewindesegment sind vorzugsweise mit einem üblichen positiven Flankenwinkel und ohne Hinterschnitt ausgebildet.

Unter einem Hinterschnitt einer Gewindeflanke im Sinne der Erfindung ist eine Ausbildung einer Gewindeflanke zu verstehen, bei der eine Gewindeflanke einen in radialer Richtung äußeren Bereich aufweist, der gegenüber einem in radialer inneren Bereich der gleichen Gewindeflanke in axialer Richtung vorsteht. Dies kann zum Beispiel der Fall sein, wenn in eine Gewindeflanke eine Hintergreifung oder Nut eingebracht ist, oder der Neigungswinkel α einer Flanke negativ ist (bei einem üblichen metrischen Gewinde ist der Neigungswinkel jeder Flanke positiv).

Infolge des Hinterschnitts sind das Außengewinde der Gewindestange und das Innengewinde des Innengewindesegments bei Eingriff ineinander miteinander verhakt. Bei einem auf die Gewindestange ausgeübten Druck, der so in axialer Richtung wirkt, dass die mit Hinterschnitt versehenen Gewindeflanken von Gewindestange und Innengewindesegment gegeneinander gedrückt werden, ist eine Verlagerung des Innengewindesegments infolgedessen in radialer Richtung nicht möglich. Das Gewinde ist quasi gesichert und gehemmt.

Die jeweils den mit Hinterschnitt versehenen Gewindeflanken gegenüberliegenden "normalen" Gewindeflanken der Gewindestange und des Innengewindesegments können im Sinne der Erfindung bei einem auf die Gewindestange wirkenden Axialdruck entsprechender Höhe wie eine schiefe Ebene oder ein Umlenkgetriebe wirken. Ist ein Axialdruck, der so in axialer Richtung wirkt, dass die ohne Hinterschnitt ausgebildeten Gewindeflanken von Gewindestange und Innengewindesegment gegeneinander gedrückt werden, ausreichend hoch, wird infolge der schräg zur Axialrichtung ausgerichteten Gewindeflanken das Innengewindesegment in radiale Richtung nach außen verlagert oder positioniert. Das Außengewinde der Gewindestange und das Innengewinde des Innengewindesegments gelangen so außer Eingriff und die Gewindestange kann in axialer Richtung durchrutschen. Auf diese Weise ist eine Schnellpositionierung der Gewindestange in axialer Richtung möglich.

Insgesamt ist durch die Erfindung eine Schnellpositionierung der Gewindestange in die eine axiale Richtung möglich, während eine reine Axialverschiebung in die andere, entgegen gerichtete axiale Richtung durch den Hinterschnitt gehemmt ist und die Gewindestange in diese Richtung nur durch Schrauben bewegt werden kann. Die Gewindestange kann durch die Erfindung ohne Schraubbewegung in axialer Richtung vorgeschoben werden kann, bis ein durch den Stab ausgeübter Gegendruck zu groß wird. Ab diesem Punkt kann eine weitere Axialpositionierung durch Schrauben erfolgen, wobei hohe Kräfte auf den Stab übertragen werden können. Dabei ist ein Lösen der Gewinde von Gewindestange und Innengewindesegment durch den Hinterschnitt in vorteilhafter Weise nicht möglich und das Instrument gesichert.

Sobald der Stab die gewünschte Endposition erreicht hat, kann dieser mittels einer Set-Screw oder Ähnlichem am Implantat gesichert werden. Zum Entfernen des Instruments ist dann zunächst die Gewindestange durch Herausschrauben aus dem Innengewindesegment soweit zu lösen, bis sie druckentlastet ist. Durch Betätigung einer bei einer besonderen Ausführungsform vorliegenden Entriegelungseinrichtung können dann, also bei druckentlasteter Gewindestange, die Gewinde von Gewindestange und Innengewindesegment trotz des Hinterschnitts außer Eingriff gebracht werden. Die Gewindestange kann dann mittels einer reinen Axialpositionierung zurückgezogen werden. Es ist ein besonderer Vorteil der Erfindung, dass neben einem weitgehend ermüdungsfreiem Arbeiten und einer Zeitersparnis, da nicht die gesamte Axialpositionierung durch Schrauben erfolgen muss, ein Entkoppeln der beiden Gewinde unter hoher axialer Drucklast nicht möglich ist, so dass Verletzungen umliegender Strukturen oder von OP-Personal vermieden und verhindert werden können.

Die vorliegende Erfindung bezieht sich auf ein Instrument gemäß dem unabhängigen Anspruch. Vorteilhafte Ausführungsformen der Erfindung sind in den Unteransprüchen beansprucht und werden nachfolgend näher erläutert.

Die Kopplungseinheit ist dazu eingerichtet, eine Kopplung des Instruments mit dem Implantat zu bewirken. Bei dem Implantat handelt es sich vorzugsweise um eine Pedikelschraube, die in einen Wirbel einer Wirbelsäule eingeschraubt ist. Vorzugsweise wird die Kopplungseinheit mit einem Pedikelschraubenkopf gekoppelt. Zum Koppeln des Instruments mit dem Implantat kann die Kopplungseinheit eine Implantataufnahme ausbilden oder aufweisen. Eine solche Implantataufnahme kann insbesondere zwei oder drei einander gegenüberliegende Koppelarme umfassen. Diese sind an ihrem jeweiligen proximalen Ende mit einer Koppelstruktur versehen. Zumindest die proximalen Enden der Koppelarme sind in radiale Richtung voneinander beabstandbar oder spreizbar, können im beabstandeten Zustand an einem Implantat angeordnet und durch Schließen der proximalen Enden der Koppelarme mit diesen gekoppelt und daran fest angeordnet werden.

Die Gewindestange kann zur direkten oder indirekten Anlage an einem in ein Implantat einzudrückenden Stab ausgebildet sein. Im letzten Fall kann sie mit einer Stabdrückereinheit oder einem Stabdrückerelement verbunden sein und über diese den Stab kontaktieren und positionieren/verlagern. Vorzugsweise sind die Gewindestange und der Stabdrücker zueinander um die Längsachse drehbar miteinander gekoppelt, so dass Drehungen der Gewindestange um ihre Längsachse beim Schrauben nicht auf den Stab übertragen werden.

Bei einer Ausführungsform kann die Stabdrückereinheit mit den Koppelarmen der Kopplungseinheit zusammenwirken, beispielsweise indem die Koppelarme durch Öffnungen in der Stabdrückereinheit geführt sind, so dass die Koppelarme bei einer Positionierung der Stabdrückereinheit in proximale Richtung automatisch geschlossen oder in im geschlossenen Zustand am Implantat gesichert werden.

Das Innengewindesegment ist vorzugsweise in Form einer Gewindeschale mit einem zur Gewindestange weisenden Innengewinde oder Innengewindeabschnitt ausgebildet. Im Rahmen der Erfindung weist das Instrument zumindest ein Innengewindesegment auf. Es ist jedoch von Vorteil, wenn es zwei beiderseits der Gewindestange einander gegenüberliegende Innengewindesegmente, die in umfänglicher Richtung um einen Winkel von 180° voneinander beabstandet sind, aufweist. Es ist außerdem im Rahmen, wenn das Instrument drei oder vier Innengewindesegmente in umfänglicher Richtung gleichmäßig voneinander beabstandete Innengewindesegmente aufweist (im Falle von drei Innengewindesegmenten betragen die Winkel jeweils 120°, im Falle von vier Innengewindesegmenten jeweils 90°).

Bei einer Ausführungsform kann eine mit Hinterschnitt ausgebildete Gewindeflanke einen gegenüber einer Normalen zur jeweiligen Gewindeachse geneigten negativen Flankenwinkel α aufweisen, der vorzugsweise in einem Bereich von ca. -10° bis ca. -1°, bevorzugter von ca. -8° bis ca. -2°, bevorzugter von ca. -6° bis ca. -3° noch bevorzugter von ca. -5° bis ca. -4° liegt. Es liegt auf der Hand, dass die Flankenwinkel der Gewindestange auf die des Innengewindesegments abgestimmt sind.

Von besonderem Vorteil ist, wenn die mit Hinterschnitt ausgebildeten Flankenwinkel der Gewindestange distalseitig und die mit einem Hinterschnitt ausgebildeten Flankenwinkel der Gewindehülse proximalseitig angeordnet sind. In diesem Fall ist ein Positionieren der Gewindestange in distale axiale Richtung bei in Eingriff stehenden Gewinden von Gewindestange und Innengewindesegment nicht möglich. Ein Lösen des Gewindeeingriffs durch eine Verlagerung des Innengewindesegments in radialer Richtung nach außen ist bei unter einer Druckbelastung stehenden Gewindestange nicht möglich, da der Hinterschnitt eine solche Bewegung sperrt. Die in Eingriff stehenden Gewinde sind gesichert. Wird jedoch die Gewindestange durch teilweises Lösen und Herausschrauben druckentlastet, kann der Gewindeeingriff trotz Hinterschnitt gelöst werden, wobei es zu einer geringen Verlagerung der Gewindestange in die proximale axiale Richtung kommt.

Bei einer Weiterbildung dieser Ausführungsform sind die einer Flanke mit Hinterschnitt jeweils gegenüberliegenden Gewindeflanken mit einem positiven Flankenwinkel β ausgebildet. Auf diese Weise ist ein Positionieren der Gewindestange in proximaler axialer Richtung auch bei in Eingriff stehenden Gewinden möglich, indem auf die Gewindestange ein ausreichend hoher axialer Druck aufgebracht wird, bei dem das Innengewindesegment über den positiven Flankenwinkel der proximalseitigen Flanken der Gewindestange sowie der distalseitigen Flanken des Innengewindesegments, die nach Art einer schiefen Ebene wirken, in radialer Richtung gegen die auf es wirkende Vorspannung nach außen gedrängt wird.

Bei einer Ausführungsform ist das Innengewindesegment in einem Gehäuse aufgenommen. Es kann insbesondere mittels einer Feder in Richtung der Gewindestange vorgespannt sein, welche Feder am oder im Gehäuse geführt und in radialer Richtung nach außen abgestützt ist.

Bei einer Ausführungsform der Erfindung weist das Instrument des Weiteren eine Entriegelungseinrichtung, vorzugsweise in Form von zwei Entriegelungselementen auf. Diese können insbesondere in tangentialer Richtung beiderseits des Innengewindesegments angeordnet sein. Sie können des Weiteren in radialer Richtung durch nutzerseitige Betätigung positionierbar sein. Die Entriegelungselemente stehen vorzugsweise jeweils über eine schiefe Ebene mit einem Innengewindesegment in Anlage. Sie sind wie die Innengewindesegmente gegenüber der Gewindestange in radialer Richtung positionierbar. Infolge der radial nach innen, also in Richtung der Gewindestange gerichteten Vorspannung der Innengewindesegmente und ihrer Anlage daran sind die Entriegelungselemente in radialer Richtung nach außen vorgespannt. Durch eine Positionierung der Entriegelungseinheit radialer Richtung nach innen, also in Richtung der Gewindestange, gegen die auf die Innengewindesegmente wirkende Vorspannung, zum Beispiel durch eine nutzerseitige Betätigung, können die Gewinde von Gewindestange und Innengewindesegment außer Eingriff gebracht werden. Voraussetzung hierfür ist, dass die vorstehend beschriebene Gewindeverriegelung durch den Hinterschnitt durch Druckentlastung der Gewindestange entriegelbar ist.

Bei einer besonderen Ausführungsform kann einem Nutzer des Instruments akustisch mitgeteilt werden, dass die Gewindestange druckentlastet ist. Dazu ist die Gewindestange bei einer Axialbewegung in proximaler Richtung reibgehemmt. Dies kann insbesondere durch Anlage der Gewindestange oder eines mit dieser axialgekoppelten Einheit, wie zum Beispiel der Kopplungseinheit, mit einem axialfesten Gehäuse geschehen. Vorzugsweise ist die Reibhemmung derart bemessen, dass beim Herausschrauben der Gewindestange aus dem Innengewindesegment deren Axialverschiebung verhindert wird, so dass das Innengewindesegment gegen seine Vorspannung in radialer Richtung nach außen deplatziert wird. Ist die Gewindestange ausreichend gelöst und wird nicht durch von der in das Implantat gedrückten Stange in axialer Richtung verschoben, schnappen die Gewindegänge des Innengewindesegments nach etwa einer Umdrehung der Gewindestange in den nächsten Gewindegang deren Außengewindes ein, wodurch ein akustisch wahrnehmbares Klicken dem Nutzer anzeigt, dass die Gewindestange druckentlastet ist.

Es ist von Vorteil zum Einschrauben einer Set-Screw in das Implantat bzw. die Pedikelschraube, wenn die Gewindestange hohl mit einem in axialer Richtung durchgehenden Durchgangskanal ausgebildet ist. Der Durchgangskanal ist derart dimensioniert, dass die Set-Screw wie auch ein Instrument zum Einschrauben der Set-Screw hindurch passen.

Man kann auch sagen, dass die Erfindung ein eingangs genanntes Instrument mit einem Rastmechanismus in verbesserter Form mit weiteren Sicherheitsfeatures betrifft. In einer Ausgangslage können Vorspannelemente, zum Beispiel in Form von Federn, zwei beispielsweise als Gewindeschalen ausgebildete Innengewindesegmente zusammendrücken. Diese können mit einer seitlichen Schräge an einer Entriegelungseinrichtung, zum Beispiel in Form von Drucktasten, anliegen und diese auseinanderschieben. Gewindeschalen und Drucktasten können seitlich in einem Gehäuse geführt sein. Nach oben und unten können sie durch Begrenzungsflächen beispielsweise des Gehäuses geführt sein.

Beim Einschieben der Gewindestange können die Gewindeschalen zurückfedern und sie danach sofort wieder umgreifen. Die Gewindeform besitzt einen Hinterschnitt. Das bewirkt, dass das Gewinde in eine Richtung durchgeschoben werden kann, während es sich in die andere Richtung verhakt. Das bewirkt den Vorteil, dass die mit dem Stabdrücker verbundene Gewindestange soweit von Hand eingeschoben werden kann, bis die vom Stab ausgeübte Gegenkraft zu groß wird. Ab diesem Zeitpunkt kann geschraubt werden, da sich die Gewindeflanken verhaken.

Nachdem das Instrument auf das Implantat aufgesetzt wurde, kann die Gewindestange (wie eben beschrieben) soweit per Hand nach unten geschoben werden, bis die Gegenkraft durch den Stab zu groß wird. Die Gewindestange kann wegen des verwendeten Hinterschnittgewindes und der Selbsthemmung des Gewindes in jeder beliebigen Lage losgelassen werden und hält trotzdem die Position. Dann kann weiter geschraubt und der Stab vollends in die Tulpe gedrückt werden.

Da der Rastmechanismus nach der Erfindung ein Hinterschnittgewinde besitzt, ist ein Auskoppeln (Ausfedern) der Gewinde, insbesondere unter axialem Druck, ausgeschlossen, denn eine höhere axiale Gegenkraft (des Stabes) resultiert in einem stärkeren Ineinandergreifen der Gewindeflanken. Des Weiteren sind die Drucktasten blockiert, da ein Bewegen aufgrund des blockierten, unter Last stehenden Hinterschnittgewindes nicht möglich ist. Die Drucktasten können so nicht aus Versehen betätigt werden.

Eine Set Screw kann durch den Rod Persuader hindurch im Schraubenbody festgeschraubt werden. Sie kann den Stab in der Schraube bzw. Tulpe halten, so dass der Rod Persuader gelöst und abgekoppelt werden kann. Um den blockierten Mechanismus zu lösen, genügt es, die Gewindestange wenige Umdrehungen entgegen der Einschraubrichtung zu drehen. Ein akustisches Signal, zum Beispiel in Form eines Klickens, kann den Operateur darauf hinweisen, dass das Instrument nicht mehr unter Last steht und er die Gewindestange durch Zusammendrücken der beiden Drucktasten gefahrlos zurückziehen kann. Das akustische Signal ist als zusätzliches Sicherheitsfeature vorgesehen sein. Es kann zum Beispiel dadurch erzeugt werden, dass eine Reibungskraft zum axialen Zurückziehen der Gewindestange im Instrument größer ist als eine durch Reibung im Gewinde entgegen gerichtete Reibkraft, wodurch sich die in Richtung der Gewindestange vorgespannten, insbesondere von Federn angedrückten, Gewindeschalen gegen die Vorspannung nach außen bewegen und das Gewinde durchrutscht kann. Erneutes Einrasten des Gewindes ist als Klicken wahrnehmbar.

Der konstruktiven Auslegung des akustischen Signals kann geschuldet sein, dass im Falle von zwei Entriegelungseinrichtungen der Operateur zwingend beide zu betätigen haben kann, um die Gewindestange zurückziehen zu können. Durch Betätigen der Drucktasten können Schrägen der Entriegelungseinrichtungen und der Gewindeschalen aneinander stoßen und die Gewindeschalen auseinander schieben, so dass das Gewinde außer Eingriff ist. Nun kann die Gewindestange wieder in die Ausgangsposition, insbesondere nach hinten oder in die distale Richtung, gezogen werden. Falls der Operateur die Gewindestange nicht zurückziehen, sondern lieber kontrolliert zurückschrauben möchte, kann er das tun, indem er die Gewindeschale mittelbar oder unmittelbar manuell betätigt und radial in Richtung der Gewindestange drückt. Diese Betätigung muss dann so lange erfolgen, wie er zurück schrauben möchte.

Der Druck auf die Gewindeschale kann zum Beispiel über einen Knopf einer Sicherheitsentriegelung erfolgen, was ein Ausrasten der Gewindeschale verhindert. Das kann entweder dadurch geschehen, dass die Federkraft, die gegen die Gewindeschale wirkt, erhöht wird, oder der Knopf gegen die Gewindeschale gedrückt wird. Da in beiden Fällen ein Ausrasten verhindert wird, kann das Gewinde nicht durchrutschen, und das Instrument kann zurück geschraubt werden. Wichtig ist, dass anders als beim Ausschrauben (nach hinten oder in distale Richtung bewegen) der Operateur beim Einschrauben (nach vorne oder in proximale Richtung bewegen), sobald der Stab genügend Gegenkraft bietet, jederzeit ohne Betätigung der Sicherheitsentriegelung zurückschrauben kann, da die Gegenkraft des Stabes die Gewindeflanken ineinander drückt und diese nicht auskoppeln können.

In einer weiteren bevorzugten Ausführungsform weist die Stabdrückereinheit einen Anschlag auf und das Instrument ein zur Kopplungseinheit axialfest angeordnetes Riegelelement zum Zusammenwirken mit dem Anschlag, das gegenüber der Gewindestange in radialer Richtung positionierbar ist und durch ein eine Radialkraft aufbringendes Vorspannelement in Richtung der Gewindestange in einen Eingriff mit dem Anschlag vorgespannt ist. In der nachfolgenden Beschreibung wird das das Riegelelement in Richtung der Gewindestange vorspannende Vorspannelement als "erstes Vorspannelement" bezeichnet, um es gegenüber ggf. weiteren Vorspannelementen von Ausführungsformen des Instruments unterscheiden zu können. Die Bezeichnung "erstes Vorspannelement" beinhaltet aber nicht, dass das Instrument zwingend ein zweites Vorspannelement oder weitere Vorspannelemente aufweist.

Die Längsachsen der Gewindestange und des Stabdrückers überdecken einander, anders ausgedrückt sind sie in axialer Richtung hintereinander angeordnet. Der Anschlag, der nach der Erfindung entweder an oder in der Gewindestange und/oder an oder in dem Stabdrücker ausgebildet sein kann, kann insbesondere in radialer Richtung nach außen vorspringen. Er ist nach einer Ausführungsform der Erfindung in Form eines radialen Versatz, einer Schulter oder einer Stufe ausgebildet. Es ist vorteilhaft, wenn er vollumfänglich umlaufend ausgebildet ist, da so ein unbeabsichtigtes Lösen der Gewindestange unabhängig von deren Drehpositionierung sichergestellt ist. Der Anschlag ist vorzugsweise auf der distalen Seite des Außengewindes ausgebildet.

Durch die Ausführungsform wird der Vorteil erzielt, dass zum einen durch den Gewindeeingriff zwischen der Gewindestange und dem Innengewindesegment bei feiner Verstellung und ggf. Selbsthemmung der Gewinde hohe Anpresskräfte auf einen in eine Tulpe einzupressenden Stab ausgeübt werden können. Zum anderen weist das Instrument eine Art Schnellverstellung auf, die durch Entkoppeln des Gewindeeingriffs zwischen der Gewindestange und dem Innengewindesegment bewirkt wird. Das Instrument ist daher nicht mühsam über den gesamten Verstellbereich durch relatives Verschrauben zu verstellen, sondern kann durch Entkoppeln rasch, einfach und unaufwändig zugestellt und geöffnet werden. Ein besonderer Vorteil besteht darin, dass anders als bei bekannten Instrumenten trotz der Schnellverstellung durch Entkoppeln von Gewindestange und Innengewindesegment ein vollständiges Lösen dieser beiden Bestandteile sicher und einfach verhindert wird, indem sichergestellt ist, dass das Riegelelement infolge seiner radialen Vorspannung mit dem Anschlag in Eingriff ist oder gelangt. Auf diese Weise wird eine Art Sicherheitsverriegelung ausgebildet. Eine vollständige Demontage ist dennoch durch nutzerseitiges Lösen des Riegelelements aus dem Eingriff mit dem Anschlag einfach und schnell möglich. Dabei ist sichergestellt, dass die vollständige Demontage nicht versehentlich, sondern stets nur beabsichtigt erfolgt.

Bei einem Einsatz des Instruments kann die Gewindestange durch Entkoppeln vom Innengewindesegment in axiale Richtung schnellpositioniert werden. Die Gewindestange kann so ohne Schraubbewegung in axialer Richtung vorgeschoben werden, bis ein durch den Stab ausgeübter Gegendruck zu groß wird. Ab diesem Punkt kann eine weitere Axialpositionierung durch Schrauben erfolgen, wobei hohe Kräfte auf den Stab übertragen werden können. Sobald der Stab die gewünschte Endposition erreicht hat, kann dieser mittels einer Set-Screw oder Ähnlichem am Implantat gesichert werden. Zum Entfernen des Instruments kann die Gewindestange zunächst durch Herausschrauben aus dem Innengewindesegment soweit gelöst werden, bis sie druckentlastet ist. Durch Entkoppeln des Außengewindes der Gewindestange vom Innengewinde des Innengewindesegments kann dann die Gewindestange mittels einer reinen Axialpositionierung zurückgezogen werden. Nach der Erfindung ist diese frei axiale Positionierbarkeit jedoch durch den Anschlag der Gewindestange und das mit diesem in Eingriff stehende oder in Eingriff gelangende Riegelelement begrenzt. Es ist ein besonderer Vorteil der Erfindung, dass neben einem weitgehend ermüdungsfreiem Arbeiten und einer Zeitersparnis, da nicht die gesamte Axialpositionierung durch Schrauben erfolgen muss, ein unbeabsichtigtes vollständiges Lösen oder Entfernen der Gewindestange aus dem Instrument nicht möglich ist.

Eine Ausführungsform der Erfindung ist dadurch gekennzeichnet, dass der Außendurchmesser (maximale Durchmesser) des Anschlags kleiner ist als der Fußdurchmesser des Außengewindes. Auf diese Weise ist sichergestellt, dass ein Eingriff zwischen dem Innengewindesegment und dem Anschlag nicht möglich ist. So kann eine Beschädigung des Innengewindes sicher vermieden werden. Des Weiteren kann vermieden werden, dass das Innengewindesegment bei einem vollständigen Entfernen der Gewindestange aus dem Instrument mit dem Anschlag in Eingriff gelangt und so eine Demontage erschwert.

Es ist von besonderem Vorteil, wenn in der Gewindestange ein axialer gewindefreier Abschnitt ausgebildet ist, dessen Außendurchmesser kleiner ist als der Fußdurchmesser des Außengewindes. Die axiale Länge eines derartigen Abschnitts kann nach einer Ausführungsform größer als die axiale Länge des Innengewindes des Innengewindesegments sein. Durch den gewindefreien Abschnitt wird eine Art Leerlaufbereich erzeugt, in dem die Gewindestange gegenüber dem Innengewindesegment verdreht werden kann, ohne dass es zu einem Vorschub in axialer Richtung kommt. Auf diese Weise kann das Instrument besonders einfach an einer Pedikelschraube angesetzt und zum Einpressen des Stabs vorbereitet werden. Der gewindefreie Abschnitt kann insbesondere auf der distalen Seite des Außengewindes ausgebildet sein. Eine weitere Ausführungsform der Erfindung sieht vor, dass sowohl distal als auch proximal ein jeweils ein gewindefreier Abschnitt ausgebildet ist.

Eine Ausführungsform der Erfindung ist dadurch gekennzeichnet, dass das Instrument ein Entriegelungselement aufweist, das mit dem Riegelelement zusammenwirkt. Das Riegelelement kann insbesondere durch eine nutzerseitige Betätigung des Entriegelungselements, unter Umständen gegen die durch das erste Vorspannelement aufgebrachte Vorspannung, in radialer Richtung nach außen bewegt werden und derart vom Anschlag entkoppelbar sein. Vorzugsweis ist das Entriegelungselement in radialer Richtung relativ zur Gewindestange positionierbar.

Nach einer weiteren Ausführungsform kann das Entriegelungselement in einem Gehäuse aufgenommen sein. Darin kann es insbesondere mittels einer Feder als erstes Vorspannelement in Richtung von der Gewindestange fort, also nach radial außen, vorgespannt sein. In einer besonderen Ausführungsform ist diese Feder zwischen Riegelelement und Entriegelungselement angeordnet und verspannt diese beiden Element gegeneinander. Eine Vorspannung des Riegelelements in Richtung der Gewindestange kann dabei bewirkt werden, indem das Entriegelungselement mit einem im Gehäuse gebildeten Anschlag zusammenwirken kann, der eine Begrenzung für eine radial von der Gewindestange fort gerichtete Bewegung des Entriegelungselements bildet. Auf diese Weise bildet das Entriegelungselement eine Art schwimmende Lagerung oder Aufnahme für das Riegelelement aus.

Es ist von besonderem Vorteil, wenn das Riegelelement eine Kontaktfläche für die Gewindestange aufweist, mit der es am Kopfdurchmesser (Außendurchmesser) des Außengewindes anliegend in axialer Richtung auf der Gewindestange gleiten kann, ohne mit dem Außengewinde in Eingriff zu gelangen. Auf diese Weise wird eine funktionale Trennung der Sicherheitsverriegelung von der Gewindeverstellung erreicht, was eine besonders einfache Bedienung des Instruments sicherstellt. Des Weiteren kann das Riegelelement eine nach Art einer schiefen Ebene angeordnete, also schräg zur Längsachse der Gewindestange ausgerichtete, Einlauffläche zum Kontakt mit dem distalen Ende der Gewindestange aufweisen, die ein Einschieben der Gewindestange in das Instrument erleichtert.

Eine weitere Ausführungsform der Erfindung ist dadurch gekennzeichnet, dass das Innengewindesegment gegenüber der Gewindestange in radialer Richtung positionierbar ist und durch ein eine Radialkraft aufbringendes zweites Vorspannelement in Richtung der Gewindestange vorgespannt ist, wobei das Innengewindesegment vorzugsweise in einem Gehäuse aufgenommen ist und vorzugsweise mittels einer Feder als zweites Vorspannelement in Richtung der Gewindestange vorgespannt ist. Das Innengewindesegment ist insbesondere durch Radialpositionierung mit dem Außengewinde der Gewindestange in Eingriff bzw. außer Eingriff bringbar. Bei in radialer Richtung zur Gewindestange positioniertem Innengewindesegment, im Folgenden auch erste Position bezeichnet, stehen das Außengewinde der Gewindestange und das Innengewinde des Innengewindesegments miteinander in Eingriff. Bei in radialer Richtung von der Gewindestange fort positioniertem Innengewindesegment, im Folgenden auch als zweite Position bezeichnet, sind sie außer Eingriff gebracht. In der zweiten Position ist daher die Gewindestange relativ zum Innengewindesegment frei axialpositionierbar, das heißt axial sowohl in distaler als auch in proximaler Richtung positionierbar, insbesondere durch nutzerseitige Einwirkung verschiebbar.

Das Innengewindesegment kann insbesondere in dem Gehäuse aufgenommen sein, in dem auch das Riegelelement aufgenommen ist sowie das Entriegelungselement gelagert ist. Es kann insbesondere mittels einer Feder in Richtung der Gewindestange vorgespannt sein, welche Feder am oder im Gehäuse geführt und in radialer Richtung nach außen abgestützt ist.

Eine Ausführungsform der Erfindung ist dadurch gekennzeichnet, dass Gewindeflanken der Gewindestange und Gewindeflanken des Innengewindesegments, die einander zugewandt sind, jeweils einen Hinterschnitt aufweisend ausgebildet sind

Nach einer Ausführungsform der Erfindung sind das Riegelelement und das Innengewindesegment auf einander diametral gegenüberliegenden Seiten der Gewindestange angeordnet. Dabei kann das Entriegelungselement durch nutzerseitige Betätigung in eine erste Richtung, insbesondere in radialer Richtung auf die Gewindestange zu, sowie in eine zweite Richtung, insbesondere in radialer Richtung von der Gewindestange fort, positionierbar sein. Auf diese Weise kann mit einem einzigen Entriegelungselement als nutzerseitige Betätigungseinheit entweder eine Entriegelung des Innengewindes des Innengewindesegments vom Außengewinde der Gewindestange oder eine Entriegelung des Riegelelements vom Anschlag bewirkt werden. Dies ist besonders nutzerfreundlich. Insbesondere kann das Entriegelungselement bei Positionierung in die erste Richtung das Innengewindesegment gegen die Radialkraft des Vorspannelements von der Gewindestange fortbewegt werden und mit deren Außengewinde außer Eingriff gebracht werden, und bei Positionierung in die zweite Richtung das Riegelelement gegen die Radialkraft des Vorspannelements von der Gewindestange fort bewegt und mit deren Anschlag außer Eingriff gebracht werden.

Durch die Erfindung können insbesondere die folgenden Vorteile erzielt werden: Zwei Drucktasten, zum bewussten Bedienen Hinterschnittgewinde, selbstsicherndes Gewinde Je höher die Kraft, desto sicherer wirkt der Mechanismus gegen Lösen Blockierte Drucktasten bei Last, kein versehentliches Lösen möglich Akustisches Signal (Klicken] bei Lastfreiheit während des Zurückdrehens Umschaltmöglichkeit um bei Lastfreiheit weiter zurückschrauben zu können

Weitere Merkmale und Vorteile der vorliegenden Erfindung ergeben sich aus der folgenden beispielhaften und nicht beschränkenden Beschreibung der Erfindung anhand von Figuren. Diese sind lediglich schematischer Natur und dienen nur dem Verständnis der Erfindung. Dabei zeigen:
Fig. 1 ein Instrument nach der Erfindung in einer perspektivischen Ansicht,
Fig. 2 eine perspektivische Ansicht eines Rastmechanismus des Instruments der Fig. 1,
Fig. 3 eine Schnittansicht des Rastmechanismus der Fig. 2 quer zur Axialrichtung,
Fig. 4 einen Ausschnitt des Rastmechanismus der Fig. 2 in einer Schittansicht in axialer Richtung,
Fig. 5 eine teilweise freigeschnittene perspektivische Ansicht des Rastmechanismus der Fig. 2,

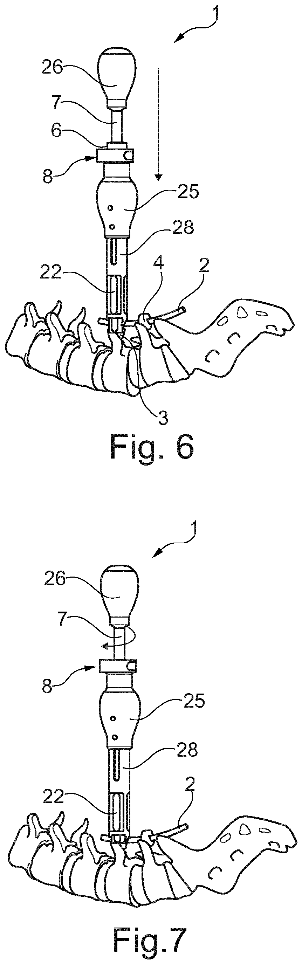

Fig. 6 eine Darstellung des Instruments beim Einsetzen eines Stabs in eine Pedikelschraube im Rahmen einer Wirbelsäulenoperation zu einem ersten Zeitpunkt und
Fig. 7 eine Darstellung des Instruments beim Einsetzen eines Stabs in eine Pedikelschraube im Rahmen einer Wirbelsäulenoperation zu einem zweiten Zeitpunkt.
Fig. 8 ein Instrument nach der Erfindung in einer perspektivischen Ansicht,
Fig. 9 eine Schnittansicht eines Rastmechanismus des Instruments der Fig. 8 in Richtung der Längsachse des Instruments,
Fig. 10 eine Schnittansicht des Rastmechanismus der Fig. 8 quer zur Axialrichtung in einer ersten Funktionsstellung,
Fig. 11 eine perspektivische teilweise freigestellte Ansicht des Rastmechanismus der Fig. 9 in der ersten Funktionsstellung,
Fig. 12 eine Schnittansicht des Rastmechanismus der Fig. 9 quer zur Axialrichtung in einer zweiten Funktionsstellung,
Fig. 13 eine perspektivische teilweise freigestellte Ansicht des Rastmechanismus der Fig. 9 in der zweiten Funktionsstellung
Fig. 14 eine Schnittansicht des Rastmechanismus der Fig.9 quer zur Axialrichtung in einer dritten Funktionsstellung,
Fig. 15 eine perspektivische teilweise freigestellte Ansicht des Rastmechanismus der Fig. 9 in der dritten Funktionsstellung,
Fig. 16 eine Schnittansicht des Rastmechanismus der Fig. 9 quer zur Axialrichtung in einer vierten Funktionsstellung,

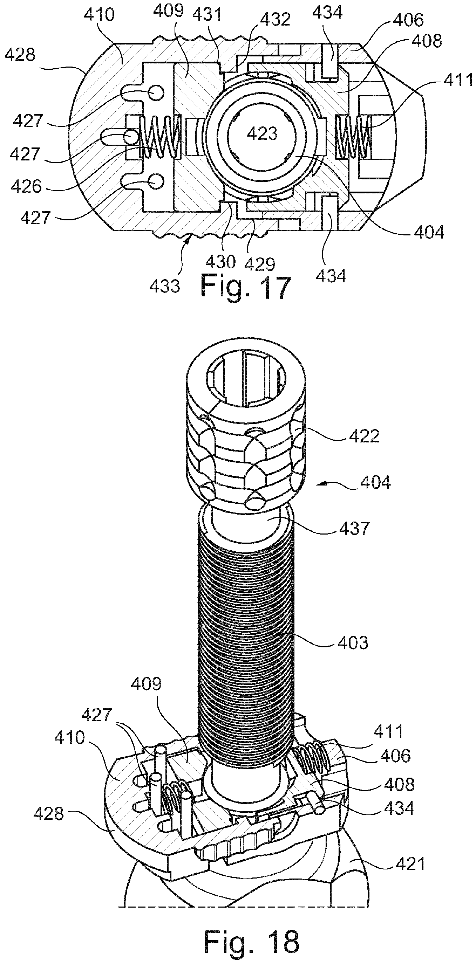

Fig. 17 eine Schnittansicht des Rastmechanismus der Fig. 9 quer zur Axialrichtung in einer fünften Funktionsstellung,
Fig. 18 eine perspektivische teilweise freigestellte Ansicht des Rastmechanismus der Fig. 9 in der fünften Funktionsstellung,
Fig. 19 eine seitliche Ansicht eines Abschnitts der Gewindestange,
Fig. 20 eine Aufsicht auf den Mechanismus des Instruments ohne Gewindestange,
Fig. 21 eine perspektivische Ansicht des Mechanismus ohne Gehäuse,

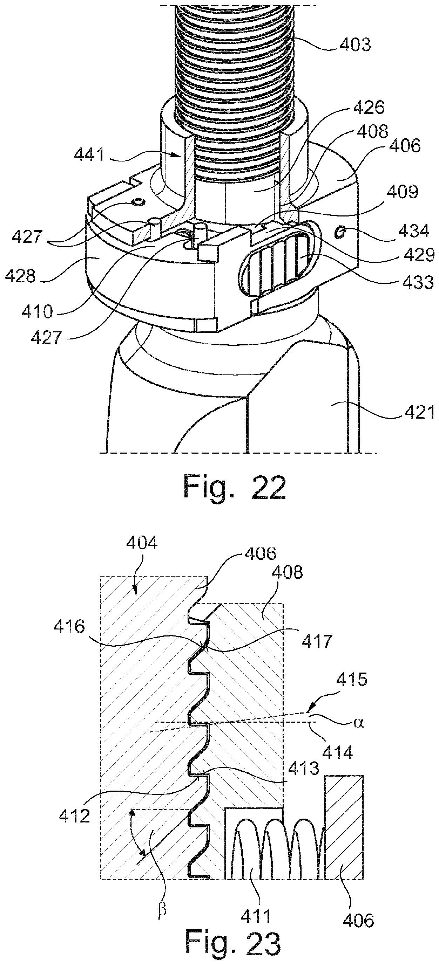

Fig. 22 die Ansicht der Fig. 8 mit teilgeschnittenem Gehäuse und
Fig. 23 einen Teilschnitt durch Innen- und Außengewinde.

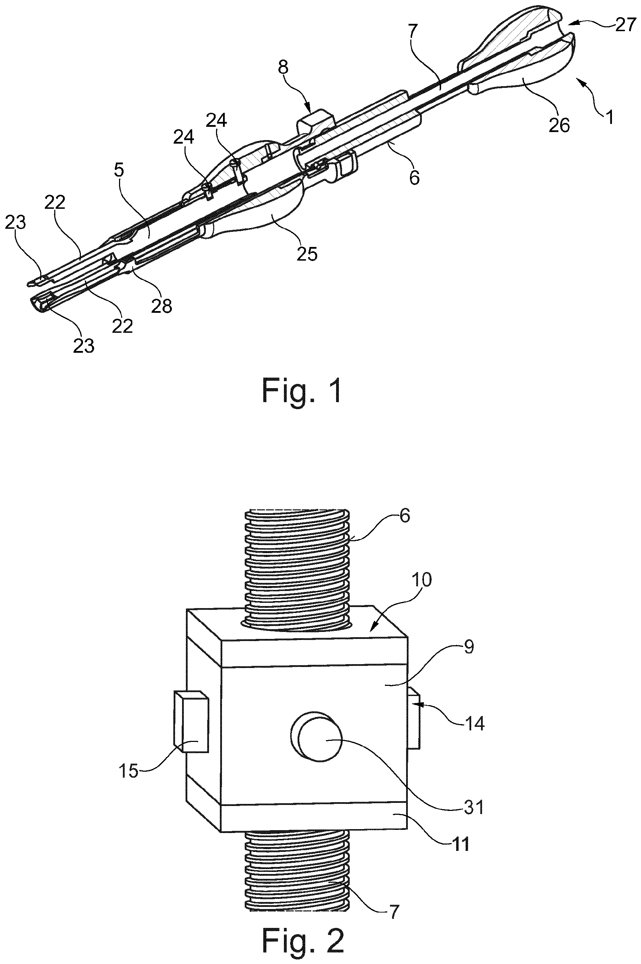

In Figur 1 ist ein erfindungsgemäßes Instrument 1 zum Relativpositionieren und/oder Führen und/oder Einführen eines Stabs 2 in eine Aufnahme eines Implantats 3, hier in einen Kopf 4 einer Pedikelschraube 3 in einer perspektivischen Ansicht dargestellt. Das Instrument umfasst im Wesentlichen eine Kopplungseinheit 5, eine mit einem Außengewinde 6 versehene und zur Kopplungseinheit 5 in axialer Richtung positionierbare Gewindestange 7 und einen zur Kopplungseinheit 5 axialfest angeordneten Rastmechanismus 8.

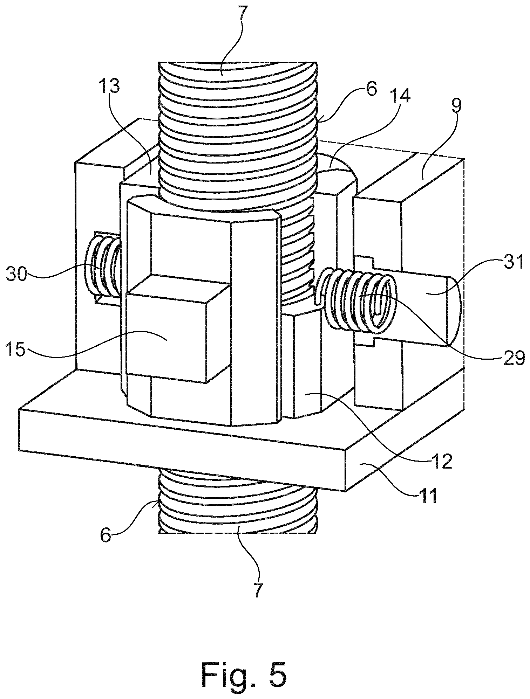

Der Rastmechanismus 8 ist zusammen mit einem Abschnitt der Gewindestange 7 in den Figuren 2, 3, 4 und 5 dargestellt. Erweist im Wesentlichen ein Gehäuse 9, einen Gehäuseboden 10, einen Gehäusedeckel 11, ein erstes Innengewindesegment 12, ein zweites Innengewindesegment 13, ein erstes Entriegelungselement 14 und ein zweites Entriegelungselement 15 auf.

Das erste wie auch das zweite Innengewindesegment 12, 13 ist jeweils gegenüber der Gewindestange 7 in radialer Richtung positionierbar und jeweils durch ein eine Radialkraft aufbringendes Vorspannelement 29, 30 in Form einer Druckfeder 29, 30 in Richtung der Gewindestange 7 vorgespannt. Infolge der radialen Positionierbarkeit kann jedes der beiden Innengewindesegmente 12, 13 durch Verschieben oder Platzieren in radialer Richtung mit dem Außengewinde 6 der Gewindestange 7 in Eingriff bzw. außer Eingriff gebracht werden.

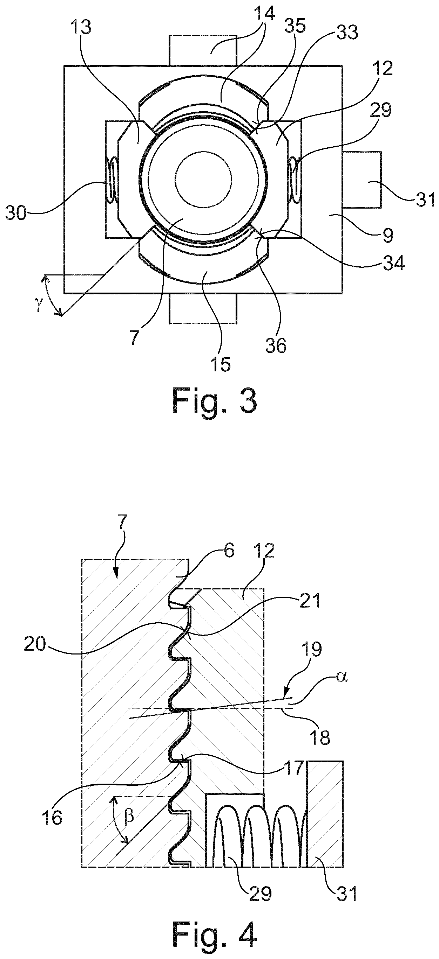

Insbesondere in Figur 4 ist dargestellt, dass Gewindeflanken 16 der Gewindestange 7 und Gewindeflanken 17 jedes Innengewindesegments 12, 13, die einander zugewandt sind, jeweils einen Hinterschnitt aufweisend ausgebildet sind. Zur Verdeutlichung des Hinterschnitts ist in Figur 4 eine Normale 18 zur Längsachse der Gewindestange 7 eingezeichnet. Ebenfalls eingezeichnet ist eine Linie 19, die sich mit den Gewindeflanken 16, 17 überdeckt. Zwischen der Normalen 18 und der Linie 19 erstreckt sich ein negativer Flankenwinkel α. Jede einer mit Hinterschnitt versehenen Gewindeflanke 16 des Außengewindes 6 der Gewindestange 7 gegenüberliegende Gewindeflanke 20 ist mit einem positiven Flankenwinkel β ausgebildet. Ebenfalls ist jede einer mit Hinterschnitt versehenen Gewindeflanke 17 jedes Innengewindesegments 12, 13 gegenüberliegende Gewindeflanke 21 mit einem positiven Flankenwinkel β ausgebildet.

Figur 1 zeigt, dass die Kopplungseinheit 5 zwei einander gegenüberliegende Koppelarme 22 aufweist, die jeweils an ihrem proximalen Ende mit einer Koppelstruktur 23 versehen sind, zur zumindest axialfesten Ankopplung des Instruments 1 am Kopf 4 einer Pedikelschraube 3. Die Kopplungseinheit 5 ist mittels Schrauben oder Nieten 24 drehfest und axialfest mit einem proximalen Griffelement 25 verbunden. Das Griffelement 25 wiederum ist drehfest und axialfest mit dem Gehäuse 9 des Rastmechanismus 8 verbunden.

Figur 2 zeigt, dass das Gehäuse 9, der Gehäuseboden 10 und der Gehäusedeckel 11 jeweils mit einer Durchgangsöffnung ausgebildet sind, in der die Gewindestange 7 angeordnet ist. Die Gewindestange 7 ist an ihrem distalen Ende mit einem distalen Griffelement 26 versehen und ihrerseits im Wesentlichen hohlzylinderförmig mit einem in Axialrichtung durchgehenden Durchgangskanal 27 versehen. An ihrem proximalen Ende ist die Gewindestange 7 axialfest mit einem Stabdrücker 28 verbunden. Die Gewindestange 7 und der Stabdrücker 28 sind relativ zueinander um die Längsachse der Gewindestange 7 drehbar.

Insgesamt kann man sagen, dass die Kopplungseinheit 5, das proximale Griffelement 25 und das Gehäuse 9, 10, 11 mit den darin aufgenommenen Innengewindesegmenten 12, 13 und Entriegelungselementen 13, 14 eine erste Einheit des Instruments 1 bilden. In ähnlicher Weise bilden die Gewindestange 7, der Stabdrücker 28 und das distale Griffelement 26 eine zweite Einheit des Instruments 1. Die erste Einheit und die zweite Einheit des Instruments sind über einen Eingriff des Außengewindes 6 der Gewindestange 7 in die Innengewinde der Innengewindesegmente 12, 13 miteinander gekoppelt.

Die Figuren 3, 4 und 5 zeigen, dass beide Innengewindesegmente 12, 13 mittels jeweils einer Feder 29 bzw. 30 in radialer Richtung nach innen, also in Richtung der Gewindestange 7 vorgespannt sind. Die Feder 29 ist über ein Betätigungselement 31 abgestützt, das eine Durchgangsöffnung im Gehäuse 9 durchgreifend in diesem aufgenommen und in radialer Richtung nach außen abgestützt ist. In ähnlicher Weise sind die Entriegelungselemente 14, 15 in Durchgangsöffnungen im Gehäuse 9 angeordnet und gehalten.

In umfänglicher Richtung ist das Innengewindesegment 12 beidseitig mit einer Anlagefläche 33, 34 versehen. Beide sind im Wesentlichen in radialer Richtung liegend ausgebildet. Die Anlagefläche 33 steht mit einer am Entriegelungselement 14 ausgebildeten entsprechenden Anlagefläche 35 in Anlage. Die Anlagefläche 34 steht mit einer am Entriegelungselement 15 ausgebildeten entsprechenden Anlagefläche 36 in Anlage. Infolge der umfänglichen Erstreckung des Innengewindesegments 12 sind die Anlageflächen 33, 34 in einem Winkel γ schräg zur Bewegungsrichtung des Innengewindesegments 12 ausgerichtet. In ähnlicher Weise sind die Anlageflächen 35, 36 schräg zur Bewegungsrichtung des jeweiligen Entriegelungselements 14, 15 ausgerichtet. Man kann also sagen, dass die Anlageflächen 33, 34, 35, 36 zwei schiefe Ebenen bilden, über die das Innengewindesegment 12 mit den Entriegelungselementen 14, 15 in Wirkverbindung steht. Das Innengewindesegment 13 ist in entsprechender Weise ausgebildet und steht in entsprechender Weise mit den Entriegelungselementen 14, 15 in Wirkverbindung.

Das Instrument 1 wird wie folgt verwendet: Zu Beginn eines Vorgangs zum Andrücken eines Stabs 2 an eine Pedikelschraube 3 ist die zweite Einheit bestehend aus distalem Griffelement 26, Gewindestange 7 und Stabdrücker 28 in distaler Richtung aus der bereits beschriebenen ersten Einheit herausgezogen. Das Instrument 1 wird in diesem Zustand mit der Koppelstruktur 23 am Kopf 4 der Pedikelschraube 3 angeordnet und gekoppelt, wobei der einzudrückende Stab 2 zwischen den beiden Koppelarmen 22 platziert ist.

Nachfolgend wird die Gewindestange 7 in proximal axiale Richtung in das Instrument 1 eingeschoben. Dies kann mit einer reinen Axialbewegung ohne Rotation der Gewindestange 7 um ihre Längsachse erfolgen. Dabei federn die Innengewindesegmente 12, 13 in radialer Richtung nach außen zurück, da die sich gegenseitig kontaktierenden Gewindeflanken 20, 21 ohne Hinterschnitt jeweils wie eine schiefe Ebene wirken. Infolge der radialen Vorspannung rutschen das Außengewinde 6 und das Innengewinde der Innengewindesegmente 12, 13 wieder ineinander, wenn eine axiale Position erreicht wird, in der Gewindegänge des Außengewindes 6 sich mit Gewindespitzen der Innengewinde überdecken.

Bei wieder ineinander greifenden Gewinden "verhaken" sich diese infolge des Hinterschnitts. Insgesamt wird durch die erfindungsgemäße Ausbildung der Gewinde bewirkt, dass die Gewindestange 7 in eine Richtung, nämlich im vorliegenden Ausführungsbeispiel in die proximale Richtung, ohne Schraubbewegung durch die Innengewindesegmente 12, 13 geschoben werden kann, während eine reine Axialbewegung in die andere Richtung (distal) verhindert wird. Das bewirkt den Vorteil, dass die mit dem Stabdrücker 28 verbundene Gewindestange 7 soweit von Hand in proximaler Richtung eingeschoben werden kann, bis die vom Stab 2 auf den Stabdrücker 28 ausgeübte Gegenkraft zu groß wird. Dann erfolgt eine weitere Zustellung in proximaler Richtung durch Einschrauben der Gewindestange 7 in die Innengewindesegmente 12, 13. Bei den vorstehend beschriebenen Vorgängen kann die Gewindestange 7 wegen des Hinterschnittgewindes und der dadurch bewirkten Selbsthemmung in jeder beliebigen Lage losgelassen werden und hält trotzdem die Position.

Da der Rastmechanismus 8 ein Hinterschnittgewinde besitzt, ist ein unbeabsichtigtes Auskoppeln des Außengewindes 6 von den Innengewinden der Innengewindesegmente 12,13, zum Beispiel durch ein ungewolltes Ausfedern gegen die Federvorspannung, ausgeschlossen, denn eine höhere axiale Gegenkraft auf den Stab 2 führt zu einem stärkeren Ineinandergreifen der Gewindeflanken. Des Weiteren sind infolge des Hinterschnitts die Entriegelungselemente 14, 15 (Drucktasten) blockiert, da sie über die Anlageflächen 33, 34, 35, 36 mit den Innengewindesegmenten 12, 13 zusammenwirken, deren Bewegen aufgrund des blockierten, unter Last stehenden Hinterschnittgewindes nicht möglich ist. Eine versehentliche Entriegelung ist somit nicht möglich.

Hat der Stab 2 im Kopf 4 der Pedikelschraube 3 seine bestimmungsgemäße Position erreicht, wird eine in den Figuren nicht gezeigte Set-Screw durch den Durchgangskanal 27 der Gewindestange 7 hindurch festgeschraubt. Sie hält den Stab 2 in der Schraube 3 bzw. im Kopf 4, so dass das Instrument nach der Erfindung nun gelöst und abgekoppelt werden kann. Dazu muss zunächst die Gewindestange 7 wenige Umdrehungen gegen die Einschraubrichtung gedreht werden.

Die Gewindestange 7 ist bei einer Axialbewegung in proximaler Richtung reibgehemmt, indem sie selbst und/oder der Stabdrücker 28 mit einem axialfesten Element des Instruments 1, zum Beispiel mit dem proximalen Griffelement 25 in Reibanlage stehen. Die Reibhemmung ist derart bemessen, dass beim Herausschrauben der Gewindestange 7 ohne zusätzliche Druckbelastung durch den Stab 2 aus den Innengewindesegmenten 12, 13 eine Axialverschiebung der Gewindestange 7 verhindert wird. Infolgedessen werden die Innengewindesegmente 12, 13 gegen die Vorspannung der Federn 29, 30 in radialer Richtung nach außen deplatziert. Wenn die Gewindestange 7 ausreichend gelöst ist und wirkt auf sie kein vom Stab 2 ausgeübter Druck mehr (was bei mittels der Set-Screw gesichertem Stab 2 der Fall ist), schnappen die Gewindegänge der Innengewindesegmente 12, 13 nach etwa einer Umdrehung der Gewindestange 7 in den nächsten Gewindegang deren Außengewindes 6 ein, wodurch ein akustisch wahrnehmbares Klicken dem Nutzer anzeigt, dass die Gewindestange 7 druckentlastet ist, und er die Gewindestange 7 durch Zusammendrücken der beiden Entriegelungselemente 14, 15 gefahrlos entriegeln und in axialer Richtung zurückziehen kann.

Zum Entriegeln muss der Operateur bei dem beschriebenen Ausführungsbeispiel zwingend beide Entriegelungselemente 14, 15 betätigen, um die Gewindestange 7 mit einer reinen Axialbewegung zurückziehen zu können. Durch Betätigen der Entriegelungselemente 14, 15 stoßen die Anlageflächen 33, 34, 35, 36 aneinander und schieben die Innengewindesegmente 12, 13 in radialer Richtung nach außen, wodurch die Gewinde außer Eingriff gebracht werden. Nun kann die Gewindestange 7 mittels rein axialer Bewegung in die Ausgangsposition gezogen werden. Falls der Operateur die Gewindestange 7 nicht zurückziehen, sondern lieber kontrolliert zurückschrauben möchte, kann er das tun, indem er das Betätigungselement 31 betätigt. Dieses muss so lange betätigt bleiben, wie er zurück schrauben möchte. Der Druck auf Betätigungselement 31 verhindert eine radial nach außen führende Bewegung der Innengewindesegmente 12, 13, im vorliegenden Beispiel durch Druck auf die Federn 29, 3ß, was deren Federkraft erhöht. Das Gewinde kann dann nicht durchrutschen und die Gewindestange 7 kann zurück geschraubt werden. Im Unterschied zum Ausschrauben (distal nach hinten bewegen) kann der Operateur beim Einschrauben (proximal nach vorne bewegen) jederzeit ohne Betätigung des Betätigungselements 31 zurückschrauben, da dann der Stab 2 genügend Gegenkraft auf die Gewindestange 7 ausübt, wodurch infolge des Hinterschnitts die Gewindeflanken 16, 17 ineinander gedrückt werden und die Gewindestange 7 nicht von den Innengewindesegmenten 12,13 entkoppelt werden kann.

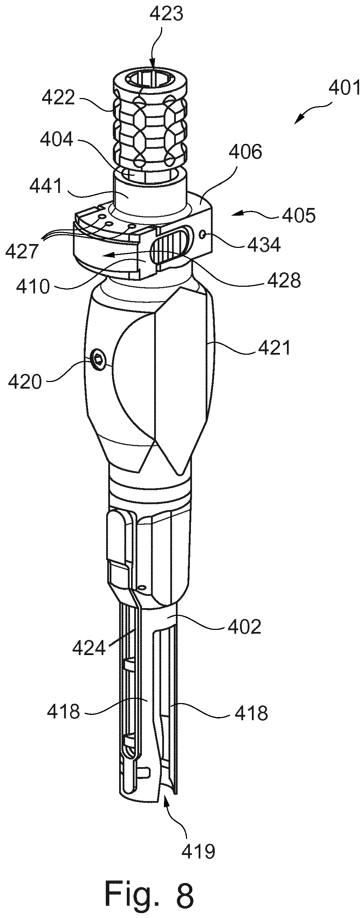

In Figur 8 ist ein erfindungsgemäßes Instrument 401 zum Relativpositionieren und/oder Führen und/oder Einführen eines in den Figuren nicht dargestellten Stabs in eine Aufnahme eines ebenfalls nicht dargestellten Implantats, wie zum Beispiel in einen Kopf einer Pedikelschraube, in einer perspektivischen Ansicht dargestellt. Das Instrument 401 umfasst im Wesentlichen eine Kopplungseinheit 402, eine mit einem Außengewinde 403 versehene und zur Kopplungseinheit 402 in axialer Richtung positionierbare Gewindestange 404 und einen zur Kopplungseinheit 402 axialfest angeordneten Rastmechanismus 405.

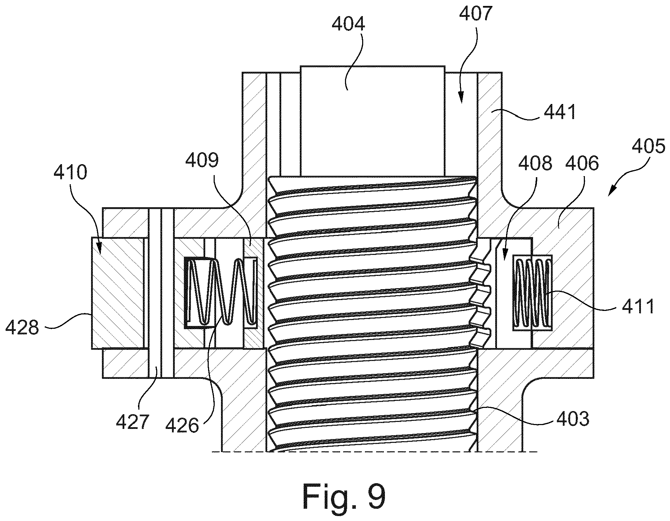

Der Rastmechanismus 405 ist zusammen mit einem Abschnitt der Gewindestange 404 vergrößert unter anderem in Figur 9 dargestellt. Er weist im Wesentlichen ein Gehäuse 406 mit einer sich in axialer Richtung erstreckenden Durchgangsöffnung 407, ein Innengewindesegment 408, ein Riegelelement 409 und ein Entriegelungselement 410 auf. Das Gehäuse 406 weist eine zylinderförmige Führung 441 auf, über die die Gewindestange 404 geführt und relativ zum Gehäuse 406 und den darin aufgenommenen und gelagerten Einheiten positioniert ist.

Das Innengewindesegment 408 ist gegenüber der Gewindestange 404 in radialer Richtung positionierbar und durch ein eine Radialkraft aufbringendes Vorspannelement 411 (zweites Vorspannelement) in Form einer Druckfeder 411 in Richtung der Gewindestange 404 vorgespannt. Infolge der radialen Positionierbarkeit kann das Innengewindesegment 408 durch Verschieben oder Platzieren in radialer Richtung mit dem Außengewinde 403 der Gewindestange 404 in Eingriff bzw. außer Eingriff gebracht werden.

Gewindeflanken 412 der Gewindestange 404 und Gewindeflanken 413 des Innengewindesegments 408, die einander zugewandt sind, sind jeweils einen Hinterschnitt aufweisend ausgebildet. Zur Verdeutlichung des Hinterschnitts, ist eine Normale 414 zur Längsachse der Gewindestange 404 eingezeichnet. Ebenfalls eingezeichnet ist eine Linie 415, die sich mit den Gewindeflanken 412, 413 überdeckt. Zwischen der Normalen 414 und der Linie 415 erstreckt sich ein negativer Flankenwinkel α. Jede einer mit Hinterschnitt versehenen Gewindeflanke 412 des Außengewindes 403 der Gewindestange 404 gegenüberliegende Gewindeflanke 416 ist mit einem positiven Flankenwinkel β ausgebildet. Ebenfalls ist jede einer mit Hinterschnitt versehenen Gewindeflanke 413 des Innengewindesegments 408 gegenüberliegende Gewindeflanke 417 mit einem positiven Flankenwinkel β ausgebildet.

Figur 8 zeigt, dass die Kopplungseinheit 402 zwei einander gegenüberliegende Koppelarme 418 aufweist, die jeweils an ihrem distalen Ende mit einer Koppelstruktur 419 versehen sind, zur zumindest axialfesten Ankopplung des Instruments 401 am Kopf einer Pedikelschraube. Die Kopplungseinheit 402 ist mittels Schrauben oder Nieten 420 drehfest und axialfest mit einem distalen Griffelement 421 verbunden. Das Griffelement 421 wiederum ist drehfest und axialfest mit dem Gehäuse 406 des Rastmechanismus 405 verbunden.

Figur 9 zeigt, dass die Gewindestange 404 in der Durchgangsöffnung 407 des Gehäuses 406 angeordnet ist. Die Gewindestange 404 ist an ihrem proximalen Ende mit einem proximalen Griffelement (in den Figuren nicht dargestellt) versehen, das über eine Aufnahme 422 mit der Gewindestange 404 drehfest gekoppelt ist, und ihrerseits im Wesentlichen hohlzylinderförmig mit einem in Axialrichtung durchgehenden Durchgangskanal 423 versehen. An ihrem distalen Ende ist die Gewindestange 404 axialfest mit einem Stabdrücker 424 verbunden. Die Gewindestange 404 und der Stabdrücker 424 sind relativ zueinander um die Längsachse der Gewindestange 404 drehbar.

Insgesamt kann man sagen, dass die Kopplungseinheit 402, das distale Griffelement 421 und das Gehäuse 406 mit dem darin aufgenommenen Innengewindesegment 408, dem Riegelelement 409, das man auch als Verriegelungsfalle bezeichnen kann, und dem Entriegelungselement 410 eine erste Einheit des Instruments 401 bilden. In ähnlicher Weise bilden die Gewindestange 404, der Stabdrücker 24 und das proximale Griffelement eine zweite Einheit des Instruments 401. Die erste Einheit und die zweite Einheit des Instruments sind über einen Eingriff des Außengewindes 403 der Gewindestange 404 in das Innengewinde 425 des Innengewindesegments 408 miteinander gekoppelt.

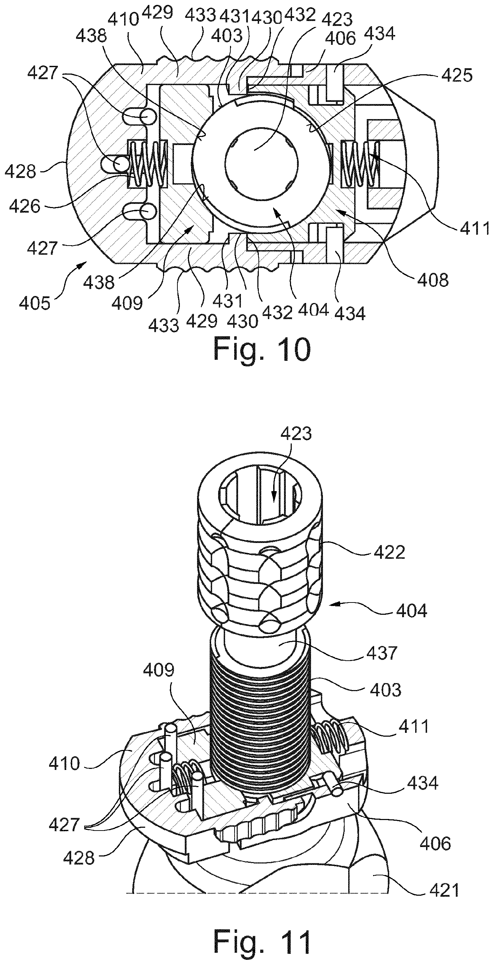

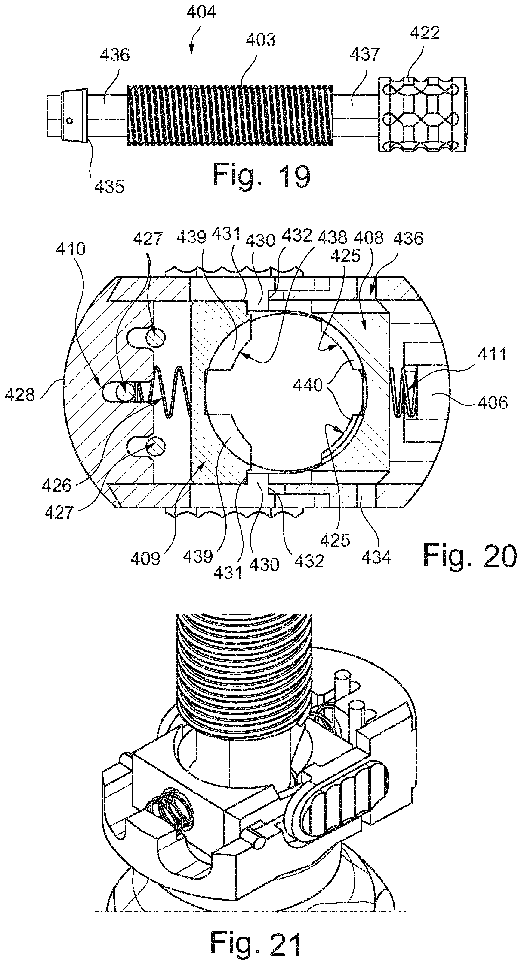

Das Innengewindesegment 408 ist mittels der Feder 411 in radialer Richtung nach innen, also in Richtung der Gewindestange 407 vorgespannt sind. Diese ist radial außen am Gehäuse 406 abgestützt. Das Riegelelement 408 ist mittels einer Druckfeder 426 (erstes Vorspannelement) in radialer Richtung gegen die Gewindestange 404 vorgespannt. Die Druckfeder 426 ist in einem Ruhezustand radial außen an einem mittleren von drei Führungstiften 427 abgestützt. Diese lagern ein Entriegelungselement 410 in radialer Richtung positionierbar im Gehäuse 406, dessen radial äußeres Ende aus dem Gehäuse 406 hinaus ragt und als Betätigungsknopf 428 ausgebildet ist. Im vorstehend genannten Ruhezustand befindet sich das Entriegelungselement 410 in radialer Richtung beabstandet von der Gewindestange 404 (Figur 10). Wird der Betätigungsknopf 428 betätigt, also radial in Richtung der Gewindestange 404 verlagert, gelangt die Druckfeder 426 mit ihm in Anlage und stützt sich daran ab. Das Entriegelungselement 410 weist, zwei seitliche Arme 429 auf, die sich beiderseits des Knopfs 428 tangential zur Gewindestange 404 erstrecken. Auf ihren der Gewindestange 404 jeweils zugewandten Seiten weisen sie jeweils einen Anschlagzapfen 430 auf, mit einer ersten Anschlagfläche 431 und einer zweiten Anschlagfläche 432. Die erste Anschlagfläche 431 ist zum Zusammenwirken mit dem Riegelelement 409 und die zweite Anschlagfläche 432 zum Zusammenwirken mit dem Innengewindesegment 408 ausgebildet. Auf den äußeren Seiten der Arme 429 ist jeweils eine Griffstruktur 433 ausgebildet (siehe zum Beispiel in Figur 10). Figur 20 zeigt, dass das Innengewindesegment 408 mit Stiften 434 im Gehäuse 406 geführt ist.

Das radial innere Ende des Riegelelements 409 ist mit zwei Kontaktflächen 438 sowie axial darüber angeordneten Gleitflächen 439 versehen. Die Kontaktflächen 438 sind derart ausgebildet, dass das Riegelelement 409 auf der äußeren Mantelfläche (Außendurchmesser) des Außengewindes 403 der Gewindestange 404 aufliegen und in axialer Richtung darauf gleiten kann, ohne mit dem Außengewinde 403 in Gewindeeingriff zu gelangen. Die Gleitflächen 439 erleichtern ein axiales Einschieben der Gewindestange 404.

Da die Gewindestange 404 während einer OP in der Regel mehrmals zurückgezogen werden muss, zum Beispiel um einen Stab in mehrere Pedikelschrauben einzupressen, ist nach der Erfindung vorgesehen, dass sich das Instrument 401 dabei nicht unbeabsichtigt zerlegt. Zu diesem Zweck ist die Gewindestange 404 mit einem Anschlag 435 ausgebildet (siehe insbesondere in Figur 19). Distal des Außengewindes 403 ist ein gewindefreier Abschnitt 436 ausgebildet. Dessen Durchmesser ist kleiner als der Fußdurchmesser (Innendurchmesser) des Außengewindes 403. Der Anschlag 435 ist distal des gewindefreien Abschnitts 436 platziert. Ein zweiter gewindefreier Abschnitt 437, der im vorliegenden Beispiel ohne Funktion ist, ist proximal des Außengewindes 403 ausgebildet.

Das Instrument 401 wird wie folgt verwendet:
Zunächst wird die Gewindestange 404 in die Durchgangsöffnung 407 des Gehäuses 406 eingeschoben. Dabei gelangt ihr distales Ende mit den Gleitflächen 439 des Riegelelements 409 in Anlage, wodurch das Riegelelement 409 gegen die Spannung der Feder 411 radial nach außen gedrängt wird. Gleiches gilt für das Innengewindesegment 408, das ähnliche Gleitflächen 440 aufweist. Die Gewindestange 404 ist nun in die Durchgangsöffnung 407 des Gehäuses 406 eingeschoben und steht mit dem Innengewindesegment 408 in Gewindeeingriff.

Mit montierter Gewindestange 404 wird in einer Ausgangssituation das Instrument 401 mit der Pedikelschraube gekoppelt. Zu Beginn eines Vorgangs zum Andrücken eines Stabs an eine Pedikelschraube ist die zweite Einheit bestehend aus proximalem Griffelement 422, Gewindestange 404 und Stabdrücker 424 in proximaler Richtung aus der bereits beschriebenen ersten Einheit herausgezogen. Das Instrument 401 wird in diesem Zustand mit der Koppelstruktur 419 am Kopf der Pedikelschraube angeordnet und gekoppelt, wobei der einzudrückende Stab zwischen den beiden Koppelarmen 418 platziert ist.

Nachfolgend wird die Gewindestange 404 in distal axiale Richtung in das Instrument 401 eingeschoben. Infolge der vorstehend beschriebenen positiven Flankenwinkel β kann die Gewindestange 404 von Hand durch reine Axialverschiebung in die distale Richtung (nach unten) gedrückt werden. Dabei wird das Innengewindesegment 408 durch die positiven Flankenwinkel β durch das Außengewinde 403 radial nach außen gedrückt, bis die Gewindestange 404 und das Innengewindesegment 408 außer Eingriff sind. Die Gewindestange kann axial verschoben werden, bis sie bzw. der Stabdrücker 424 an der Pedikelschraube anliegt. Infolge der radialen Vorspannung durch die Feder 411 wird der Gewindeeingriff wieder hergestellt. Eine weitere Axialpositionierung erfolgt nun durch Verdrehen der Gewindestange 404 und Herausschrauben aus dem Innengewindesegment 408.

Bei wieder ineinander greifenden Gewinden "verhaken" sich diese infolge des Hinterschnitts. Insgesamt wird durch den Hinterschnitt der Gewinde bewirkt, dass die Gewindestange 404 in eine Richtung, nämlich im vorliegenden Ausführungsbeispiel in die distale Richtung, ohne Schraubbewegung durch das Gehäuse geschoben werden kann (mit Verschieben des Innengewindesegments 408 nach radial außen), während eine reine Axialbewegung in die andere Richtung (proximal) verhindert wird. Das bewirkt den Vorteil, dass die mit dem Stabdrücker 424 verbundene Gewindestange 404 soweit von Hand in distaler Richtung eingeschoben werden kann, bis die vom Stab auf den Stabdrücker 424 ausgeübte Gegenkraft zu groß wird. Dann erfolgt eine weitere Zustellung in distaler Richtung durch Einschrauben der Gewindestange 404. Bei den vorstehend beschriebenen Vorgängen kann die Gewindestange 404 wegen des Hinterschnittgewindes und der dadurch bewirkten Selbsthemmung in jeder beliebigen Lage losgelassen werden und hält trotzdem die Position.

Da der Rastmechanismus 402 ein Hinterschnittgewinde besitzt, ist ein unbeabsichtigtes Auskoppeln des Außengewindes 403 vom Innengewinde des Innengewindesegments 408, zum Beispiel durch ein ungewolltes Ausfedern gegen die Federvorspannung, ausgeschlossen, denn eine höhere axiale Gegenkraft auf den Stab führt zu einem stärkeren Ineinandergreifen der Gewindeflanken. Des Weiteren ist infolge des Hinterschnitts das Entriegelungselemente 410 über eine Anlage von Anschlagzapfen 430 und Anschlagflächen 431 blockiert, da es darüber mit dem Innengewindesegment 8 zusammenwirkt, dessen Bewegen aufgrund des blockierten, unter Last stehenden Hinterschnittgewindes nicht möglich ist. Eine versehentliche Entriegelung ist somit nicht möglich.

Hat der Stab durch Verschrauben der Gewindestange 404 gegenüber dem Innengewindesegment 408 im Kopf der Pedikelschraube seine bestimmungsgemäße Position erreicht, wird eine in den Figuren nicht gezeigte Set-Screw durch den Durchgangskanal 423 der Gewindestange 404 hindurch festgeschraubt. Sie hält den Stab in der Schraube bzw. im Kopf, so dass das Instrument 401 nun gelöst und abgekoppelt werden kann. Dazu muss zunächst die Gewindestange 404 wenige Umdrehungen gegen die Einschraubrichtung gedreht werden.

Die Gewindestange 404 ist bei einer Axialbewegung in distaler Richtung reibgehemmt, indem sie selbst und/oder der Stabdrücker 424 mit einem axialfesten Element des Instruments 401, zum Beispiel mit dem distalen Griffelement 421 in Reibanlage stehen. Die Reibhemmung ist derart bemessen, dass beim Herausschrauben der Gewindestange 404 ohne zusätzliche Druckbelastung durch den Stab aus dem Innengewindesegment 408 eine Axialverschiebung der Gewindestange 404 verhindert wird. Infolgedessen wird das Innengewindesegment 408 gegen die Vorspannung der Feder 411 in radialer Richtung nach außen deplatziert. Wenn die Gewindestange 404 ausreichend gelöst ist und wirkt auf sie kein vom Stab ausgeübter Druck mehr (was bei mittels der Set-Screw gesichertem Stab der Fall ist), schnappen die Gewindegänge des Innengewindesegments 408 nach etwa einer Umdrehung der Gewindestange 404 in den nächsten Gewindegang des Außengewindes 403 ein, wodurch ein akustisch wahrnehmbares Klicken dem Nutzer anzeigt, dass die Gewindestange 404 druckentlastet ist, und er die Gewindestange 404 durch Druckbetätigung des Entriegelungselements 410, 428 gefahrlos entriegeln und in axialer Richtung zurückziehen kann.

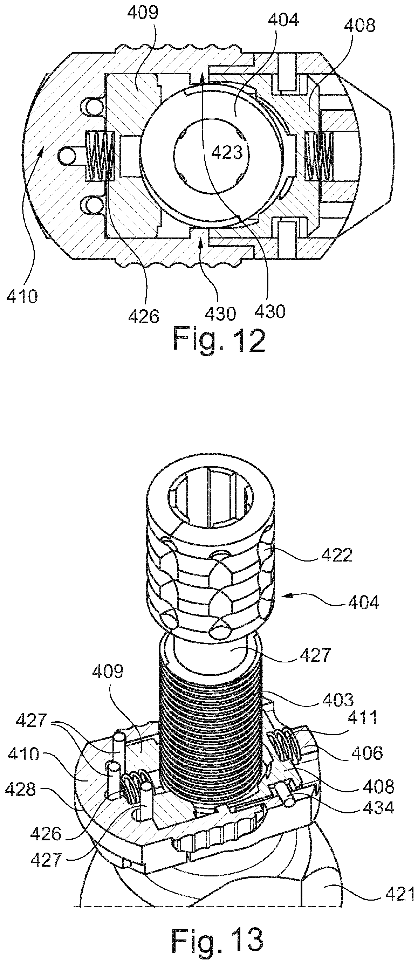

Zum Entriegeln muss der Operateur bei dem beschriebenen Ausführungsbeispiel den Knopf 428 des Entriegelungselements 410 betätigen, um die Gewindestange 404 mit einer reinen Axialbewegung zurückziehen zu können. Der Zustand bei gedrücktem Knopf 428 zum Entriegeln der Gewinde ist in den Figuren 12 und 13 dargestellt. Durch drücken des Knopfs 428 verlagert sich das Betätigungselement 410 radial zur Gewindestange 404 hin. Seine Anschlagzapfen 430 gelangen dabei mit den Anschlagflächen 432 in Anlage, so dass das Innengewindesegment 408 gegen die Radialspannung der Feder 411 nach radial außen fort von der Gewindestange 404 gedrängt wird. Auf diese Weise werden die Gewinde außer Eingriff gebracht. Die Gewindestange 404 kann dann mittels rein axialer Bewegung in die Ausgangsposition, also in die proximale Richtung, zurückgezogen werden.

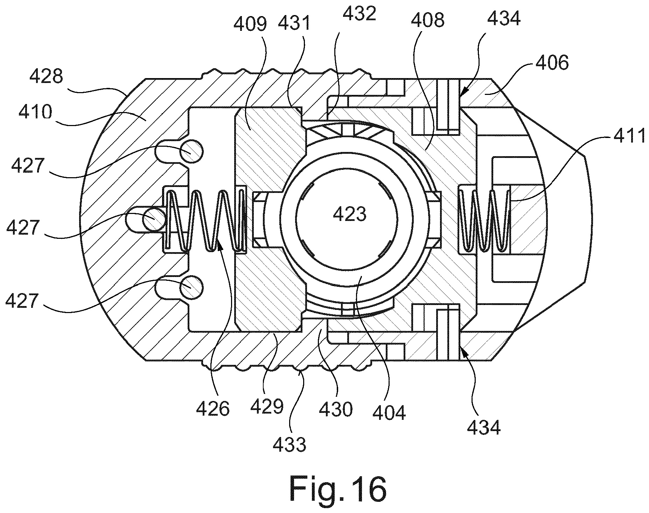

Die Gewindestange 404 kann aufgrund der erfindungsgemäßen Demontagesicherung aber nur soweit zurückgezogen werden, bis das Riegelelement 409 mit dem Anschlag 435 der Gewindestange 404 in Anlage gelangt. Durch die radial nach innen wirkende Vorspannung des Riegelelements 409 mittels der am Betätigungselement abgestützten Feder 426 wird das Riegelelement 409 fortwährend in Richtung der Gewindestange 404 gedrängt. Dabei gleitet es mit seinen Kontaktflächen 438 auf dem Außendurchmesser des Außengewindes 403 ab, bis die Gewindestange in axialer Richtung derart relativplatziert ist, dass das Riegelelement 409 mit dem gewindefreien Abschnitt 436 in Überdeckung gelangt. Durch seine radiale Vorspannung schnappt das Riegelelement 409 radial nach innen (quasi in den gewindefreien Abschnitt hinein) und gelangt bei weiterer Axialbewegung der Gewindestange 404 mit dem Anschlag 435 in Anlage. Die Gewindestange ist dann nicht weiter in proximale Richtung axialbewegbar, so dass das Instrument 401 vor einer unbeabsichtigten vollständigen Demontage der Gewindestange 404 gesichert ist. Die vorgespannte Feder 426 aus dem vorangegangenen Schritt begünstigt das sichere Einschnappen. Selbst wenn der Knopf 428 jetzt losgelassen wird (siehe Figur 16) ist der Mechanismus aufgrund der fortwährenden Vorspannung der Feder 426 so abgestimmt, das das Verriegelungselement 409 weiterhin in den gewindefreien Abschnitt 436 eingreift, mit dem Anschlag 435 der Gewindestange 404 in Anlage ist und ein unbeabsichtigtes Zerlegen verhindert wird. Aus Figur 16 ist auch der Zweck der Stifte 434 zu erkennen: Damit bei losgelassenem Knopf 426 das Innengewindesegment 408 nicht in den gewindefreien Abschnitt 436 eingreift, wird es von den Stiften 434 radial außen zurück gehalten.

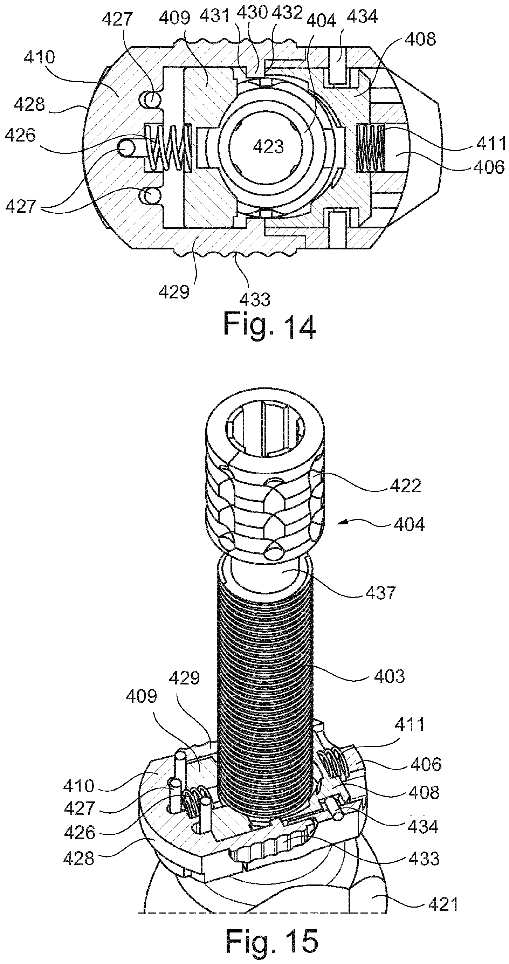

Nachdem die Gewindestange 404 zurückgezogen wurde, kann das Instrument 401 auf eine andere Pedikelschraube aufgesetzt werden und die Anwendung wird wie vorstehend beschrieben wiederholt, bis der Stab in alle Pedikelschrauben hineingedrückt und fixiert wurde. Die während einer OP stattfindenden Anwendungszyklen sind in den Figuren 10 bis 16 dargestellt.

Nach dem OP-Ende kann das Instrument 1 trotz der Demontagesicherung zum Reinigen zerlegt werden. Dazu wird das Betätigungselement 10 an den seitlich dafür vorgesehenen geriffelten Griffstrukturen 33 radial nach außen, also von der Gewindestange 4 fort, gezogen. Dies bewirkt, dass die Anschlagzapfen 30 mit den Anschlagflächen 31 des Riegelelements 9 in Anlage kommen und bei fortwährender Radialpositionierung das Riegelelement 9 radial nach außen bewegt wird, bis dieses vom Anschlag 35 der Gewindestange 4 gelöst ist. Die Gewindestange 4 ist dann vollständig freigegeben und kann durch reine Axialbewegung vollständig aus dem Gehäuse 6 entfernt werden.

Da das Außengewinde 403 einen größeren Durchmesser aufweist als der gewindefreie Abschnitt 436, kann das Innengewindesegment 408 nicht in den Hinterschnitt schnappen würde.

### Bezugszeichenliste

- 1: Instrument
- 2: Stab
- 3: Implantat, Pedikelschraube
- 4: Kopf
- 5: Kopplungseinheit
- 6: Außengewinde
- 7: Gewindestange
- 8: Rastmechanismus
- 9: Gehäuse
- 10: Gehäuseboden
- 11: Gehäusedeckel
- 12: Innengewindesegment
- 13: Innengewindesegment
- 14: Entriegelungselement
- 15: Entriegelungselement
- 16: Gewindeflanke
- 17: Gewindeflanke
- 18: Normal zur Längsachse
- 19: Linie
- 20: Gewindeflanke
- 21: Gewindeflanke
- 22: Koppelarme
- 23: Koppelstruktur
- 24: Schraube, Niet
- 25: proximales Griffelement
- 26: distales Griffelement
- 27: Durchgangskanal
- 28: Stabdrücker
- 29: Feder
- 30: Feder
- 31: Betätigungselement
- 33: Anlagefläche
- 34: Anlagefläche
- 35: Anlagefläche
- 36: Anlagefläche
- 401: Instrument
- 402: Kopplungseinheit
- 403: Außengewinde
- 404: Gewindestange
- 405: Rastmechanismus
- 406: Gehäuse
- 407: Durchgangsöffnung
- 408: Innengewindesegment
- 409: Riegelelement
- 410: Entriegelungselement
- 411: zweite Vorspannelement, Druckfeder
- 412: Gewindeflanke
- 413: Gewindeflanke
- 414: Normal zur Längsachse
- 415: Linie
- 416: Gewindeflanke
- 417: Gewindeflanke
- 418: Koppelarme
- 419: Koppelstruktur
- 420: Schraube, Niet
- 421: distales Griffelement
- 422: proximales Griffelement
- 423: Durchgangskanal
- 424: Stabdrücker
- 425: Innengewinde
- 426: erste Vorspannelement Druckfeder
- 427: Führungsstift
- 428: Knopf
- 429: Arm
- 430: Anschlagzapfen
- 431: Anschlagfläche
- 432: Anschlagfläche
- 433: Griffstruktur
- 434: Stift für Innengewindesegment
- 435: Anschlag der Gewindestange
- 436: gewindefreier Abschnitt
- 437: gewindefreier Abschnitt
- 438: Kontaktfläche des Riegelelements für Gewindestange
- 439: Gleitfläche der Riegelelements
- 440: Gleitfläche des Innengewindesegments
- 441: Führung

- α: negativer Flankenwinkel
- β: positiver Flankenwinkel
- γ: Winkel der Anlageflächen

## Patentansprüche

1. Instrument (1, 401) zum Führen eines Stabs (2) in eine Aufnahme (4) eines Implantats (3), insbesondere einer Pedikelschraube (3), umfassend
eine Kopplungseinheit (5, 402) zum Koppeln des Instruments (1, 402) mit dem Implantat (3), insbesondere mit einem Kopf (4) der Pedikelschraube (3),
eine mit einem Außengewinde (6, 403) versehene und zur Kopplungseinheit (5, 402) in axialer Richtung positionierbare Gewindestange (7, 404) und
zumindest ein zur Kopplungseinheit (5) axialfest angeordnetes Innengewindesegment (12, 13, 408), das gegenüber der Gewindestange (7, 404) in radialer Richtung positionierbar ist und durch ein eine Radialkraft aufbringendes Vorspannelement (29, 30, 411) in Richtung der Gewindestange (7, 404) vorgespannt ist,
wobei das Innengewindesegment (12, 13, 408) durch Radialpositionierung mit dem Außengewinde (6, 403) der Gewindestange (7, 404) in Eingriff bzw. außer Eingriff bringbar ist,
**dadurch gekennzeichnet, dass**
Gewindeflanken (16, 416) der Gewindestange (7, 404) und Gewindeflanken (17, 417) des Innengewindesegments (12, 13, 408), die einander zugewandt sind, jeweils einen Hinterschnitt aufweisend ausgebildet sind, und
wobei wenigstens ein Entriegelungselement (14, 15, 410) in tangentialer Richtung des Innengewindesegments (12, 13, 408) angeordnet und in radialer Richtung durch nutzerseitige Betätigung positionierbar ist.

2. Instrument (1, 401) nach Anspruch 1, **dadurch gekennzeichnet, dass** die mit Hinterschnitt ausgebildeten Gewindeflanken (16, 17, 416, 417) einen gegenüber einer Normalen zur jeweiligen Gewindeachse geneigten negativen Flankenwinkel α aufweisen, der vorzugsweise in einem Bereich von ca. -10° bis ca. -1°, bevorzugter von ca. -8° bis ca. -2°, bevorzugter von ca. -6° bis ca. -3° noch bevorzugter von ca. -5° bis ca. -4° liegt.

3. Instrument (1, 401) nach Anspruch 1 oder 2, **dadurch gekennzeichnet, dass** die mit Hinterschnitt ausgebildeten Flankenwinkel (16, 416) der Gewindestange (7, 404) distalseitig und die mit einem Hinterschnitt ausgebildeten Flankenwinkel (17, 417) des Innengewindesegments (12, 13, 408) proximalseitig angeordnet sind.

4. Instrument (1, 401) nach einem der vorstehenden Ansprüche, **dadurch gekennzeichnet, dass** das Innengewindesegment (12, 13, 408) in einem Gehäuse (9, 10, 11, 406) aufgenommen ist und mittels einer Feder (29, 30, 411) in Richtung der Gewindestange (7, 404) vorgespannt ist.

5. Instrument (1) nach einem der vorstehenden Ansprüche, **dadurch gekennzeichnet, dass** das Instrument (1) zwei Innengewindesegmente (12, 13) aufweist, die beiderseits der Gewindestange (7) radial einander gegenüberliegend angeordnet sind.

6. Instrument (1) nach einem der vorstehenden Ansprüche, **dadurch gekennzeichnet, dass** zwei Entriegelungselemente (14, 15) in tangentialer Richtung beiderseits des Innengewindesegments (12, 13) angeordnet und in radialer Richtung durch nutzerseitige Betätigung positionierbar sind.

7. Instrument (1) nach Anspruch 6, **dadurch gekennzeichnet, dass** die Entriegelungselemente (14, 15) jeweils über eine schiefe Ebene mit dem Innengewindesegment (12, 13) in Anlage stehen.

8. Instrument (1, 401) nach einem der vorhergehenden Ansprüche, **dadurch gekennzeichnet, dass** die Gewindestange (7, 404) bei einer Axialbewegung in proximaler Richtung reibgehemmt ist, insbesondere durch Anlage mit einem axialfesten Gehäuse (25, 421).

9. Instrument (1, 401) nach Anspruch 8, **dadurch gekennzeichnet, dass** die Reibhemmung derart bemessen ist, dass beim Herausschrauben der Gewindestange (7, 404) aus dem Innengewindesegment (12, 13, 408) dieses gegen seine Vorspannung in radialer Richtung nach außen deplatziert wird.

10. Instrument (1, 401) nach einem der vorhergehenden Ansprüche, **dadurch gekennzeichnet, dass** die Gewindestange (7, 404) hohl mit einem in axialer Richtung durchgehenden Durchgangskanal (28, 423) ausgebildet ist.

11. Instrument (401) nach einem der vorgehenden Ansprüche, des Weiteren aufweisend eine Stabdrückeinheit mit einem Anschlag (435) und ein zum Rastmechanismus (405) axialfest angeordnetes Riegelelement (409) zum Zusammenwirken mit dem Anschlag (435), das gegenüber der Gewindestange (404) in radialer Richtung positionierbar ist und durch ein eine Radialkraft aufbringendes Vorspannelement (411) in Richtung der Gewindestange (404) in einen Eingriff mit dem Anschlag (435) vorgespannt ist.

12. Instrument (401) nach Anspruch 11, des Weiteren aufweisend ein Entriegelungselement (410), das um das Innengewindesegment (408) oder um das Riegelelement (409) herum angeordnet ist und das Innengewindesegment (408) und/oder das Riegelelement (409) in radialer Richtung verschieben kann.

13. Instrument (401) nach Anspruch 11 und 12, **dadurch gekennzeichnet, dass** das Entriegelungselement (410), wenigstens einen Anschlagzapfen (430) aufweist, vorzugsweise zwei gegenüberliegende, der das Innengewindesegment (408) oder das Riegelelement (409) in radialer Richtung auswärts durch nutzerseitige Betätigung bewegt.

14. Instrument (401) nach einem der vorstehenden Ansprüche 11 bis 13, **dadurch gekennzeichnet, dass** der Außendurchmesser des Anschlags (435) kleiner ist als der Fußdurchmesser des Außengewindes (403)

15. Instrument (401) nach einem der Ansprüche 11 bis 14, **dadurch gekennzeichnet, dass** das Riegelelement (409) eine glatte Fläche aufweist, die der Gewindestange (404) zugewandt ist.

## Claims

1. An instrument (1, 401) for guiding a rod (2) into a holder (4) of an implant (3), in particular of a pedicle screw (3), comprising
a coupling unit (5, 402) for coupling the instrument (1, 402) to the implant (3), in particular to a head (4) of the pedicle screw (3),
a threaded rod (7, 404) provided with a male thread (6, 403) and positionable in the axial direction relative to the coupling unit (5, 402), and
at least one internally threaded segment (12, 13, 408) arranged in an axially fixed manner relative to the coupling unit (5), wherein the segment can be positioned in radial direction relative to the threaded rod (7, 404) and is prestressed toward the threaded rod (7, 404) by a prestressing element (29, 30, 411) applying a radial force,
wherein the internally threaded segment (12, 13, 408) can be brought into engagement with, and disengaged from the male thread (6, 403) of the threaded rod (7, 404) by radial positioning,
**characterized in that**
thread flanks (16, 416) of the threaded rod (7, 404) and thread flanks (17, 417) of the internally threaded segment (12, 13, 408), which face each other, are each formed with an undercut, and
wherein at least one unlocking element (14, 15, 410) is arranged in the tangential direction of the internally threaded segment (12, 13, 408) and is positionable in the radial direction by user actuation.

2. The instrument (1, 401) according to claim 1, **characterized in that** the thread flanks (16, 17, 416, 417) formed with an undercut have a negative flank angle α inclined relative to a normal to the respective thread axis, said angle being preferably in a range of approx. -10° to approx. -1°, more preferably from approx. -8° to approx. -2°, more preferably from approx. -6° to approx. -3°, even more preferred from approx. -5° to approx. -4°.

3. The instrument (1, 401) according to claim 1 or 2, **characterized in that** the flank angles (16, 416) of the threaded rod (7, 404) which are formed with an undercut are arranged distally and the flank angles (17, 417) of the internally threaded segment (12, 13, 408) which are formed with an undercut are arranged proximally.

4. The instrument (1, 401) according to any of the preceding claims, **characterized in that** the internally threaded segment (12, 13, 408) is received in a housing (9, 10, 11, 406) and is prestressed toward the threaded rod (7, 404) by means of a spring (29, 30, 411).

5. The instrument (1) according to any of the preceding claims, **characterized in that** the instrument (1) has two internally threaded segments (12, 13) which are arranged radially opposite one another on both sides of the threaded rod (7).

6. The instrument (1) according to any of the preceding claims, **characterized in that** two unlocking elements (14, 15) are arranged in tangential direction on both sides of the internally threaded segment (12, 13) and are positionable in radial direction by user-side actuation.

7. The instrument (1) according to claim 6, **characterized in that** the unlocking elements (14, 15) are in contact with the internally threaded segment (12, 13) in each case via an inclined plane.

8. The instrument (1, 401) according to any of the preceding claims, **characterized in that** the threaded rod (7, 404) is frictionally inhibited during an axial movement in the proximal direction, in particular by contact with an axially fixed housing (25, 421).

9. The instrument (1, 401) according to claim 8, **characterized in that** the frictional inhibition is dimensioned such that when the threaded rod (7, 404) is unscrewed from the internally threaded segment (12, 13, 408), it is displaced outwards in the radial direction against its preload.

10. The instrument (1, 401) according to any of the preceding claims, **characterized in that** the threaded rod (7, 404) is formed to be hollow with a passage channel (28, 423) continuously extending in the axial direction.

11. The instrument (401) according to any of the preceding claims, further comprising a rod pushing unit with a stop (435) and a locking element (409) arranged in an axially fixed manner relative to the latching mechanism (405) for cooperating with the stop (435), which can be positioned in radial direction with respect to the threaded rod (404) and is prestressed toward the threaded rod (404) into engagement with the stop (435) by a prestressing element (411) applying a radial force.

12. The instrument (401) according to claim 11, further comprising an unlocking element (410) disposed around the internally threaded segment (408) or around the locking element (409) and capable of displacing the internally threaded segment (408) and/or the locking element (409) in radial direction.

13. The instrument (401) according to claim 11 and 12, **characterized in that** the unlocking element (410) has at least one stop pin (430), preferably two opposite stop pins, which moves the internally threaded segment (408) or the locking element (409) outwards in radial direction by user-side actuation.

14. The instrument (401) according to any of the preceding claims 11 to 13, **characterized in that** the outer diameter of the stop (435) is smaller than the base diameter of the male thread (403).

15. The instrument (401) according to any of claims 11 to 14, **characterized in that** the locking element (409) has a smooth surface facing the threaded rod (404).

## Revendications

1. Instrument (1, 401) pour le guidage d'une tige (2) dans un logement (4) d'un implant (3), en particulier d'une vis pédiculaire (3), comprenant
une unité de couplage (5, 402) pour le couplage de l'instrument (1, 402) avec l'implant (3), en particulier avec une tête (4) de la vis pédiculaire (3),
une tige filetée (7, 404) pourvue d'un filet extérieur (6, 403) et positionnable dans le sens axial par rapport à l'unité de couplage (5, 402) et
au moins un segment à filet intérieur (12, 13, 408) agencé de manière fixe axialement par rapport à l'unité de couplage (5) qui peut être positionné par rapport à la tige filetée (7, 404) dans le sens radial et est précontraint par un élément de précontrainte (29, 30, 411) appliquant une force radiale en direction de la tige filetée (7, 404),
dans lequel le segment à filet intérieur (12, 13, 408) peut être amené en prise ou hors prise par positionnement radial avec le filet extérieur (6, 403) de la tige filetée (7, 404),
**caractérisé en ce que**
des flancs de filet (16, 416) de la tige filetée (7, 404) et des flancs de filet (17, 417) du segment à filet intérieur (12, 13, 408) qui sont tournés l'un vers l'autre, sont réalisés présentant respectivement une contre-dépouille, et
dans lequel au moins un élément de déverrouillage (14, 15, 410) est agencé dans le sens tangentiel du segment à filet intérieur (12, 13, 408) et peut être positionné dans le sens radial par actionnement côté utilisateur.

2. Instrument (1, 401) selon la revendication 1, **caractérisé en ce que** les flancs de filet (16, 17, 416, 417) réalisés avec contre-dépouille présentent un angle de flanc α négatif incliné par rapport à une normale à l'axe de filet respectif qui se trouve de préférence dans une plage d'environ -10° à environ -1°, plus préférentiellement d'environ -8° à environ -2°, plus préférentiellement d'environ -6° à environ -3°, encore plus préférentiellement d'environ -5° à environ -4°.

3. Instrument (1, 401) selon la revendication 1 ou 2, **caractérisé en ce que** les angles de flanc (16, 416) réalisés avec contre-dépouille de la tige filetée (7, 404) sont agencés côté distal et les angles de flanc (17, 417) réalisés avec une contre-dépouille du segment à filet intérieur (12, 13, 408) sont agencés côté proximal.

4. Instrument (1, 401) selon l'une quelconque des revendications précédentes, **caractérisé en ce que** le segment à filet intérieur (12, 13, 408) est reçu dans un boîtier (9, 10, 11, 406) et est précontraint au moyen d'un ressort (29, 30, 411) en direction de la tige filetée (7, 404).

5. Instrument (1) selon l'une quelconque des revendications précédentes, **caractérisé en ce que** l'instrument (1) présente deux segments à filet intérieur (12, 13) qui sont agencés à l'opposé radialement l'un à l'autre des deux côtés de la tige filetée (7).

6. Instrument (1) selon l'une quelconque des revendications précédentes, **caractérisé en ce que** deux éléments de déverrouillage (14, 15) sont agencés dans le sens tangentiel des deux côtés du segment à filet intérieur (12, 13) et peuvent être positionnés dans le sens radial par actionnement côté utilisateur.

7. Instrument (1) selon la revendication 6, **caractérisé en ce que** les éléments de déverrouillage (14, 15) sont en appui respectivement par le biais d'un plan oblique avec le segment à filet intérieur (12, 13).

8. Instrument (1, 401) selon l'une quelconque des revendications précédentes, **caractérisé en ce que** la tige filetée (7, 404) est inhibée en friction lors d'un mouvement axial dans le sens proximal, en particulier par appui avec un boîtier fixe axialement (25, 421).

9. Instrument (1, 401) selon la revendication 8, **caractérisé en ce que** l'inhibition de friction est dimensionnée de telle manière que, lors du dévissage de la tige filetée (7, 404) hors du segment à filet intérieur (12, 13, 408), celui-ci soit déplacé vers l'extérieur dans le sens radial contre sa précontrainte.

10. Instrument (1, 401) selon l'une quelconque des revendications précédentes, **caractérisé en ce que** la tige filetée (7, 404) est réalisée de manière creuse avec un canal débouchant (28, 423) débouchant dans le sens axial.

11. Instrument (401) selon l'une quelconque des revendications précédentes, présentant en outre une unité de pression de tige avec une butée (435) et un élément de verrou (409) agencé de manière fixe axialement par rapport au mécanisme d'encliquetage (405) pour la coopération avec la butée (435), lequel peut être positionné par rapport à la tige filetée (404) dans le sens radial et est précontraint par un élément de précontrainte (411) appliquant une force radiale en direction de la tige filetée (404) dans une prise avec la butée (435).

12. Instrument (401) selon la revendication 11, présentant en outre un élément de déverrouillage (410) qui est agencé autour du segment à filet intérieur (408) ou autour de l'élément de verrou (409) et peut déplacer le segment à filet intérieur (408) et/ou l'élément de verrou (409) dans le sens radial.

13. Instrument (401) selon les revendications 11 et 12, **caractérisé en ce que** l'élément de déverrouillage (410) présente au moins un tenon de butée (430), de préférence deux opposés, qui déplace le segment à filet intérieur (408) ou l'élément de verrou (409) dans le sens radial vers l'extérieur par actionnement côté utilisateur.

14. Instrument (401) selon l'une quelconque des revendications précédentes 11 à 13, **caractérisé en ce que** le diamètre extérieur de la butée (435) est inférieur au diamètre de pied du filet extérieur (403).

15. Instrument (401) selon l'une quelconque des revendications 11 à 14, **caractérisé en ce que** l'élément de verrou (409) présente une surface lisse qui est tournée vers la tige filetée (404).
